# EUROPEAN PATENT APPLICATION

(11) **EP 4 216 153 A1**
(43) Date of publication of application: **26.07.2023**
(21) Application number: 21869283.8
(22) Date of filing: 09.09.2021
(51) Int. Cl.: G06T 7/00, A61B 5/00, A61B 5/107

(54) **SKIN SURFACE ANALYSIS DEVICE AND SKIN SURFACE ANALYSIS METHOD**

(30) Priority: 17.09.2020 JP 2020156214
(71) Applicant: Hiroshima University, Higashihiroshima-shi Hiroshima 739-8511 (JP); Kawasaki Gakuen Educational Foundation, Kurashiki-shi, Okayama 701-0192 (JP)
(72) Inventor: KOIDE Tetsushi, Higashihiroshima-shi, Hiroshima 739-8511 (JP); HIDE Michihiro, Higashihiroshima-shi, Hiroshima 739-8511 (JP); AOYAMA Yumi, Kurashiki-shi, Okayama 701-0192 (JP)
(74) Representative: BRP Renaud & Partner mbB Rechtsanwälte Patentanwälte Steuerberater
(86) International application number: PCT/JP2021/033184
(87) International publication number: WO 2022/059596

(57) **Abstract**

Local image enhancement processing is executed on an image obtained by imaging a transcription material. The enhanced image is divided into a plurality of patch images and input to a machine learning identifier. The patch images after segmentation output from the machine learning identifier are combined to generate a likelihood map image of skin ridges from the whole image based on a result of the segmentation. Binarization processing is executed on the likelihood map image to generate a binary image. A skin ridge region is extracted based on the binary image to calculate the area of the skin ridge region.

## Description

### TECHNICAL FIELD

The present disclosure relates to a skin surface analysis device and a skin surface analysis method for analyzing a human skin surface.

### BACKGROUND ART

On the surface of the human skin (skin surface), there are grooves called skin folds and areas called skin ridges bordered by skin folds. In human skin, a minute amount of sweat droplets are secreted under the resting condition. This sweating secreted at rest is called basal sweating. It is known that basal sweating is secreted mainly at the skin folds, correlates with skin surface hydration, and plays an important role in maintaining the skin's barrier function. Inflammatory skin diseases, such as atopic dermatitis, cholinergic urticaria, prurigo, and lichen amyloidosis, may develop and/or worsen their symptoms by a decrease in the barrier function of the skin due to a basal sweating disturbance. Therefore, the detection method of the patient's basal perspiration would be useful for diagnosis and treatment, as it would provide information for determining the treatment plan.

The impression mold technique (IMT or IM method) is a method for detecting basal sweating, and quantitates sweating function. Dental silicone impression material was applied to the skin surface to form a film after a few minutes. Peeled silicone impression material from the skin transcribes skin surface microstructure and sweating state.

### CITATION LIST

### PATENT DOCUMENT

Patent Document 1: Domestic Republication No. 2018-230733 of PCT International Application

### SUMMARY OF THE INVENTION

### TECHNICAL PROBLEM

The IMT allows precise transcription of a skin surface microstructure to a silicone material in the form of a film, thereby making it possible to identify skin ridges and measure the area of the skin ridges. The IMT also allows precise transcription of sweat droplets to the silicone material, thereby making it possible to measure the number, diameters, and areas of the sweat droplets. Accordingly, the conditions of the skin surface can be analyzed. Use of this analysis result is advantageous in quantitatively grasping the tendency of an atopic dermatitis patient, for example, having a larger area of skin ridges and a smaller number of sweat droplets than a healthy person.

IMT allows skin ridges and sweat droplets to be distinguished based on an enlarged image of a transcription surface of the silicone material. Specifically, a magnified image of the transcription surface of the silicone material, which is magnified by an optical microscope, is obtained and displayed on a monitor. While viewing the image on the monitor, an inspector identifies skin ridges and skin folds, surrounds and colors the portions corresponding to the skin ridges, and calculates the area of the colored portions. The inspector finds out sweat droplets, colors the portions corresponding to the sweat droplets, and calculates the area of the colored portions. This procedure allows a quantitative grasp of the conditions of the skin surface but has the following problems.

That is, it is not only that a skin surface microstructure is complicated, but also that the skin surface microstructure greatly differs depending on skin diseases from which a patient suffers. Thus, it takes time for an inspector, during distinguishing, to determine where in an image is skin folds or skin ridges, and a limited number of samples can be processed within a certain time. In addition, silicone may contain bubbles which are hardly distinguished from the sweat droplets; thus, distinguishing the sweat droplets is also time and labor consuming work. In addition to a longer time needed for the work of distinguishing between skin folds and skin ridges and distinguishing sweat droplets, there is also a problem of individual variations, such as different distinguishing results due to different abilities of inspectors in making determinations by viewing an image. Longer work may lead to overlooking or other problems.

Even a single person has different numbers of sweat droplets from part to part of the skin surface. An analysis result may be inappropriate unless a part with an average number of sweat droplets is set to be a measurement target. In order to grasp such an average part, distinguishing between the skin folds and the skin ridges and distinguishing the sweat droplets need to be made in a wide range of the skin surface, which is a factor that further increases the time required for the analysis.

The present disclosure was made in view of these problems. It is an objective of the present disclosure to improve the accuracy in analyzing the conditions of a skin surface and to reduce the time required for the analysis.

### SOLUTIONS TO THE PROBLEMS

In order to achieve the objective, a first aspect of the present disclosure is directed to a skin surface analysis device for analyzing a skin surface, using a transcription material to which a human skin surface microstructure is transcribed, the skin surface analysis device including: an image input section to which an image obtained by imaging the transcription material is input; a local image enhancement processor configured to execute local image enhancement processing of enhancing contrast of a local region of the image input to the image input section to generate an enhanced image; a patch image generator configured to divide, into a plurality of patch images, the enhanced image generated by the local image enhancement processor; a machine learning identifier configured to receive the patch images generated by the patch image generator and execute segmentation of each of the patch images received; a whole image generator configured to generate a whole image by combining the patch images segmented and output from the machine learning identifier; a likelihood map generator configured to generate a likelihood map image of skin ridges based on a result of the segmentation from the whole image generated by the whole image generator; a binarization processor configured to execute binarization processing on the likelihood map image generated by the likelihood map generator to generate a binary image; a region extractor configured to extract a skin ridge region based on the binary image generated by the binarization processor; and a skin ridge analyzer configured to calculate an area of the skin ridge region extracted by the region extractor.

According to this configuration, local image enhancement processing is executed on an input image of the transcription material to which a human skin surface microstructure has been transcribed, to generate the enhanced image. This improves the visibility of the details of the image. The image before executing the local image enhancement processing may be a color image or a grayscale image. The enhanced image is divided into a plurality of patch images, each of which is then input to the machine learning identifier and segmented. The segmentation technique for each patch image is a known deep learning technique. This segmentation determines, for example, a category to which each pixel belongs, and categorizes the pixels into a skin ridge, a skin fold, a sweat droplet, and others. A whole image is generated by combining the patch images segmented and output from the machine learning identifier. From the whole image, a likelihood map image of skin ridges is generated based on a result of segmentation. A binary image is generated from the likelihood map image. In a case, for example, where white represents a skin ridge region, a skin ridge region can be distinguished by extracting a white region. The skin surface can be analyzed by calculating the area of the extracted skin ridge region.

In second and third aspects of the present disclosure, the skin surface analysis device may further include: a likelihood map generator configured to generate a likelihood map image of sweat droplets based on a result of the segmentation from the whole image generated by the whole image generator; a sweat droplet extractor configured to extract the sweat droplets based on the likelihood map image generated by the likelihood map generator; and a sweat droplet analyzer configured to calculate a distribution of the sweat droplets extracted by the sweat droplet extractor.

According to this configuration, a whole image is generated by combining the patch images segmented and output from the machine learning identifier. From the whole image, a likelihood map image of sweat droplets is generated based on a result of segmentation. In a case, for example, where white in the likelihood map image represents a sweat droplet, a sweat droplet can be distinguished by extracting a white region. The skin surface can be analyzed by calculating a distribution of the extracted sweat droplets.

In a fourth aspect of the present disclosure, the transcription material is obtained by an impression mold technique, and the skin surface analysis device further includes a grayscale processor configured to convert an image obtained by imaging the transcription material to grayscale.

The IMT allows precise transcription of the skin surface using silicone, which further improves the analysis accuracy. The silicone may be colored in pink, for example. However, according to this configuration, the image of the transcription material is converted to grayscale by the grayscale processor, thereby making it possible to handle the image as a grayscale image suitable for analysis. Accordingly, the processing speed can be increased.

In a fifth aspect of the present disclosure, the patch image generator may generate the patch images so that adjacent ones of the patch images partially overlap each other.

That is, if an image is divided into a plurality of patch images without overlapping adjacent patch images, an edge of a skin ridge or a sweat droplet may happen to overlap the boundary between the adjacent patch images, which may degrade the accuracy in distinguishing the skin ridge or the sweat droplet overlapping the boundary. By contrast, according to this configuration, the adjacent patch images partially overlap each other, allowing a skin ridge or a sweat droplet to be accurately distinguished even at the position described above.

In a sixth aspect of the present disclosure, an input image and an output image of the machine learning identifier may have a same resolution. This configuration allows accurate output of the shape of fine skin ridges and the size of a sweat droplet, for example.

In a seventh aspect of the present disclosure, the skin ridge analyzer sets, on an image, a plurality of grids in a predetermined size and calculates a ratio between the skin ridge region and a skin fold region in each of the grids.

According to this configuration, if, for example, the fineness of a skin surface needs to be evaluated, the fineness of the skin surface can be evaluated based on the ratio between the skin ridge region and the skin fold region in the grid set on a binary image or a grayscale image. A ratio of the skin ridge region equal to or higher than a predetermined value can be used as an index for determining a coarse skin, whereas a ratio of the skin ridge region lower than the predetermined value can be used as an index for determining a fine skin.

In an eighth aspect of the present disclosure, the skin ridge analyzer may convert the ratio between the skin ridge region and the skin fold region in each of the grids into numbers to obtain a frequency distribution (histogram).

In a ninth aspect of the present disclosure, the region extractor may determine, after extracting the skin ridge region, whether each portion of the skin ridge region extracted is raised and divides the skin ridge region by a portion determined to be unraised.

That is, in some state of a disease, there may be a groove formed in a part of a skin ridge. In this case, an unraised portion, that is, a recess is present in the skin ridge region extracted. The skin ridge region divided by this recess is expected to be used for determination on the state of a disease and clinical evaluation.

In a tenth aspect of the present disclosure, the skin surface analysis device further includes an information output section configured to generate and output information on a shape of the skin ridge region extracted by the region extractor. Each piece of information can thus be presented to healthcare practitioners, for example, for use in making a diagnosis or other purposes.

### ADVANTAGES OF THE INVENTION

As described above, the present disclosure allows generation of a likelihood map image of a skin surface, using a machine learning identifier, and allows a skin ridge region and sweat droplets to be distinguished, using the likelihood map image. It is therefore possible to eliminate individual variations in analysis and improve the accuracy in analyzing the conditions of the skin surface, and reduce the time required for the analysis.

### BRIEF DESCRIPTION OF THE DRAWINGS

FIG. 1 is a schematic diagram illustrating a configuration of a skin surface analysis device according to an embodiment of the present invention.
FIG. 2 is a block diagram of the skin surface analysis device.
FIG. 3 is a flowchart for explaining a flow of a first half of a skin surface analysis method.
FIG. 4 is a flowchart for explaining a flow of a second half of the skin surface analysis method.
FIG. 5A is a diagram for explaining background art and shows how to distinguish skin ridges by IMT and measure the area of the skin ridges.
FIG. 5B is a diagram for explaining background art and shows how to distinguish sweat droplets by IMT and measure the number, diameters, and areas of the sweat droplets.
FIG. 6 shows an example grayscale image.
FIG. 7 shows an example image after local image enhancement processing.
FIG. 8 shows the image after the local image enhancement processing, which is divided into a plurality of patch images.
FIG. 9 shows example segmentation by a machine learning identifier.
FIG. 10 shows an example whole image of skin ridges and skin folds.
FIG. 11 shows an example whole image of sweat droplets.
FIG. 12 shows an example likelihood map image of skin ridges and skin folds.
FIG. 13 shows an example likelihood map image of sweat droplets.
FIG. 14 shows an example image obtained by binarizing a likelihood map of skin ridges and skin folds.
FIG. 15 shows an example image obtained by extracting skin ridges and skin folds.
FIG. 16 shows an example image obtained by extracting sweat droplets.
FIG. 17 shows an example image showing comparison between the positions of sweat droplets and the skin ridges and skin folds.
FIG. 18 shows an example image where sweat droplets in skin ridges and skin folds are identified.
FIG. 19 is a histogram showing skin ridge information.
FIG. 20 shows an example heat map image of sweat droplets.
FIG. 21 shows an example skin ridge region image.
FIG. 22 is a table showing specifications of a skin ridge region.
FIG. 23 is a graph showing a two-dimensional distribution of skin ridges and skin folds per grid.
FIG. 24 shows an example image for analysis with a plurality of grids set.
FIG. 25 shows an example image obtained by combining imaging regions of nine fields of view.
FIG. 26 is a graph showing the ratio between skin ridges and skin folds on a forearm of a healthy person with a grid in a size of 100 × 100 pixels.
FIG. 27 is a graph showing the ratio between skin ridges and skin folds on a forearm of a healthy person with a grid in a size of 150 × 150 pixels.
FIG. 28 is a graph showing the ratio between skin ridges and skin folds on a forearm of a healthy person with a grid in a size of 200 × 200 pixels.
FIG. 29 is a graph showing the ratio between skin ridges and skin folds on a thigh of an atopic dermatitis patient with a grid in a size of 250 × 250 pixels.
FIG. 30 is a graph showing the ratio between skin ridges and skin folds on a thigh of an atopic dermatitis patient with a grid in a size of 100 × 100 pixels.
FIG. 31 is a graph showing the ratio between skin ridges and skin folds on a thigh of an atopic dermatitis patient with a grid in a size of 150 × 150 pixels.
FIG. 32 is a graph showing the ratio between skin ridges and skin folds on a thigh of an atopic dermatitis patient with a grid in a size of 200 × 200 pixels.
FIG. 33 is a graph showing the ratio between skin ridges and skin folds on a thigh of an atopic dermatitis patient with a grid in a size of 250 × 250 pixels.
FIG. 34 is a graph showing the ratio between skin ridges and skin folds on the forehead of an atopic dermatitis patient with a grid in a size of 100 × 100 pixels.
FIG. 35 is a graph showing the ratio between skin ridges and skin folds on the forehead of an atopic dermatitis patient with a grid in a size of 150 × 150 pixels.
FIG. 36 is a graph showing the ratio between skin ridges and skin folds on the forehead of an atopic dermatitis patient with a grid in a size of 200 × 200 pixels.
FIG. 37 is a graph showing the ratio between skin ridges and skin folds on the forehead of an atopic dermatitis patient with a grid in a size of 250 × 250 pixels.
FIG. 38 is a graph showing the ratio between skin ridges and skin folds on an elbow of an atopic dermatitis patient with a grid in a size of 100 × 100 pixels.
FIG. 39 is a graph showing the ratio between skin ridges and skin folds on an elbow of an atopic dermatitis patient with a grid in a size of 150 × 150 pixels.
FIG. 40 is a graph showing the ratio between skin ridges and skin folds on an elbow of an atopic dermatitis patient with a grid in a size of 200 × 200 pixels.
FIG. 41 is a graph showing the ratio between skin ridges and skin folds on an elbow of an atopic dermatitis patient with a grid in a size of 250 × 250 pixels.

### DESCRIPTION OF EMBODIMENT

An embodiment of the present invention will be described in detail below with reference to the drawings. The following description of a preferred embodiment is a mere example in nature, and is not intended to limit the present invention, its application, or its use.

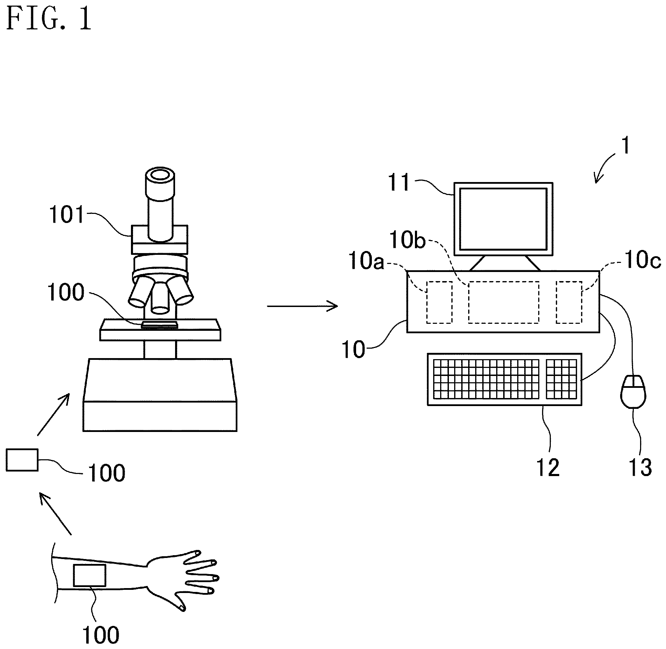

FIG. 1 is a schematic diagram illustrating a configuration of a skin surface analysis device 1 according to an embodiment of the present invention. The skin surface analysis device 1 analyzes a skin surface using a transcription material 100 to which a human skin surface microstructure is transcribed. With the use of the skin surface analysis device 1, a skin surface analysis method according to the present invention can be executed.

A case will be described in this embodiment where a skin surface is analyzed using the transcription material 100 acquired by the IMT. A human skin surface microstructure may be however transcribed to the transcription material 100 by a method other than the IMT.

The IMT is a method for detecting basal sweating, and quantitates sweating function. Dental silicone impression material was applied to the skin surface to form a film after a few minutes. Peeled silicone impression material from the skin transcribes skin surface microstructure and sweating state. The IMT has been typically used as a method of detecting basal sweating, and a detailed description thereof will thus be omitted. The dental silicone impression material may be colored pink, for example.

FIG. 1 illustrates a case where the silicone is applied over a forearm, left for a predetermined time, cured, and then peeled off from the skin to obtain the transcription material 100. The body part is however not limited thereto. The skin surface microstructure of any part, such as a leg, the chest, the back, or the forehead, may be transcribed to the transcription material 100. The IMT allows identification of skin ridges and measurement of the area of the skin ridges, since a skin surface microstructure is precisely transcribed to a silicone material in the form of a film. The IMT further allows measurement of the number, diameters, and areas of sweat droplets, since the sweat droplets are also precisely transcribed to the silicone material.

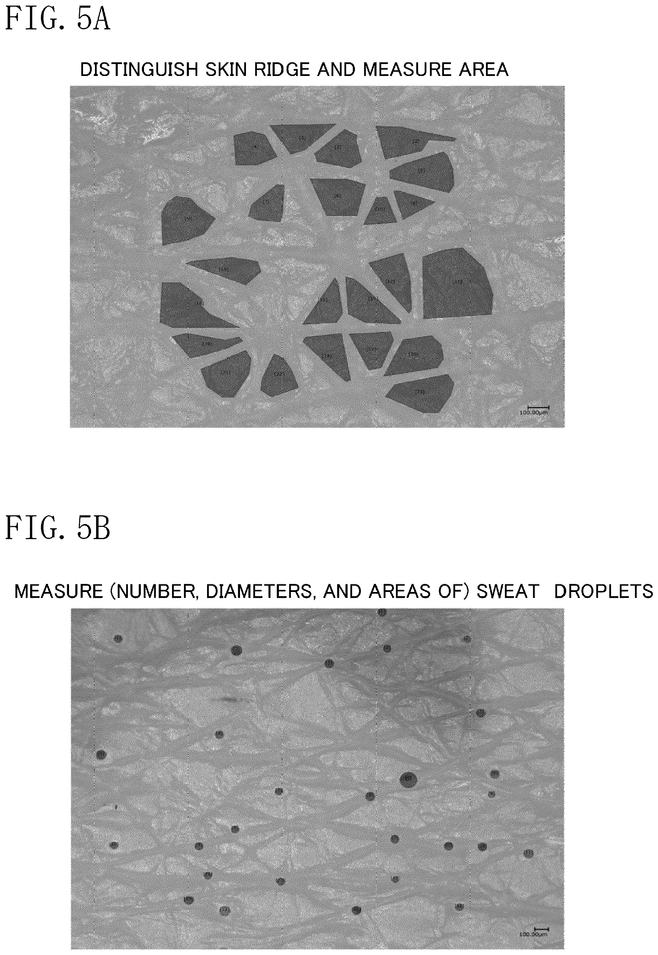

FIG. 5A is a diagram for explaining background art and shows how to distinguish skin ridges by IMT and measure the area of the skin ridges. This figure is based on an image obtained by imaging a transcription surface of the transcription material 100 magnified by a reflective stereo microscope 101 (shown in FIG. 1). An inspector displays this image on a monitor and distinguish between a skin ridge region and a skin fold region using color depths and brightness as a clue. The area of skin ridges can be obtained by measuring the area of a figure drawn by surrounding a region distinguished as a skin ridge region.

On the other hand, FIG. 5B is a diagram for explaining background art and shows how to distinguish sweat droplets by IMT and measure the number, diameters, and areas of the sweat droplets. In this figure as well, an inspector uses an image obtained by imaging a transcription surface of the transcription material 100 magnified by the reflective stereo microscope 101, displays this image on a monitor, and distinguishes sweat droplets using color depths, brightness, and shapes as a clue. The sweat droplets are marked with circles. The sweat droplets on the skin ridges and the sweat droplets in the skin folds are marked in different colors to be distinguished from each other. Accordingly, the number, diameters, and areas of the sweat droplets can be measured. Since silicone may contain bubbles, a portion substantially in a circular shape with a diameter of 40 µm or less, for example, is distinguished as a bubble.

An inspector distinguishes a skin fold, a skin ridge, and sweat droplets from one another in this manner. However, it is not only that a skin surface microstructure is complicated as shown in FIG. 5A and 5B, but also that the skin surface microstructure greatly differs depending on skin diseases from which a patient suffers. Thus, it takes time for the inspector to determine where in the image is skin ridges or skin folds, and a limited number of samples can be processed within a certain time. In addition, silicone may contain bubbles which are hardly distinguished from the sweat droplets; thus, distinguishing the sweat droplets is also time and labor consuming work.

The skin surface analysis device 1 according to this embodiment allows generation of a likelihood map image of a skin surface, even based on images such as those shown in FIGS. 5A and 5B, using a machine learning identifier 24, which will be described later, making it possible to distinguish a skin ridge region and sweat droplets using the likelihood map image. This improves the accuracy in analyzing the conditions of the skin surface and reduces the time required for the analysis.

Now, a configuration of the skin surface analysis device 1 will be described in detail. As shown in FIG. 1, the skin surface analysis device 1 can be a personal computer, for example, and includes a main body 10, a monitor 11, a keyboard 12, and a mouse 13. For example, the skin surface analysis device 1 can be obtained by installing programs for executing controls, image processing, arithmetic processing, and statistical processing, which will be described later, on a general-purpose personal computer. Alternatively, the skin surface analysis device 1 may be dedicated hardware with the programs.

The monitor 11 displays various images, user interface images for setting, or other images, and can be a liquid crystal display, for example. The keyboard 12 and the mouse 13 are those typically used as operation means for a personal computer or other devices. In place of or in addition to the keyboard 12 and the mouse 13, a touch panel or other input means may be provided. The main body 10, the monitor 11, and the operation means may be integrated.

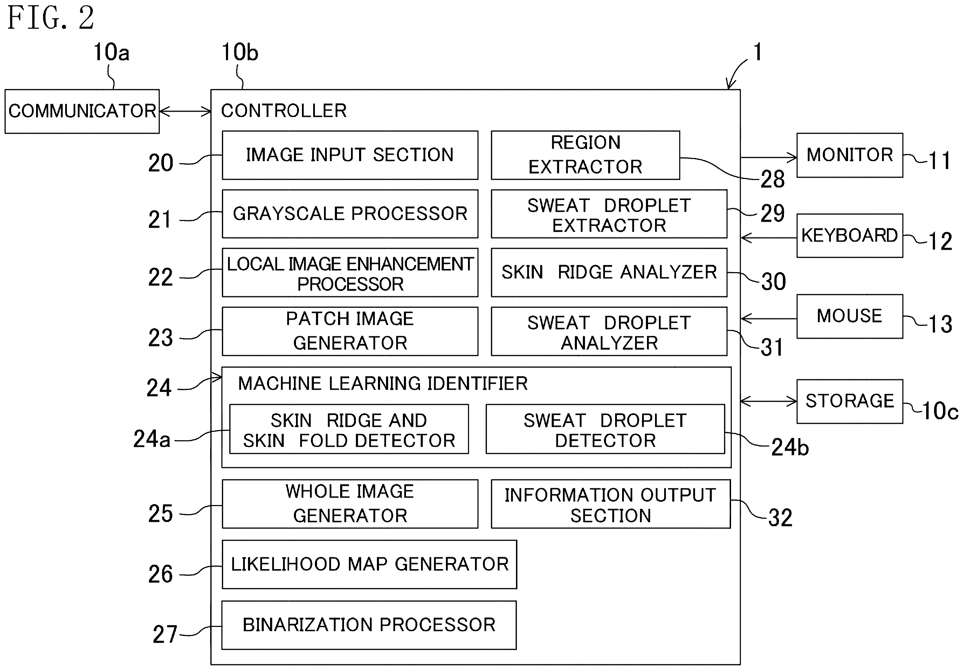

As shown in FIG. 2, the main body 10 includes a communicator 10a, a controller 10b, and a storage 10c. The communicator 10a is a section that executes data exchange with the outside and includes various communication modules, for example. Connection via the communicator 10a to a network line, such as the Internet, allows reading data from the outside and sending out data from the main body 10. The storage 10c includes a hard disk and a solid-state drive (SSD), for example, and can store various images, setting information, analysis results, statistical processing results, and the like. The storage 10c may be an external storage device or what is called a "cloud server" or a "cloud storage", for example.

Although not shown, the controller 10b can be a system LSI, an MPU, a GPU, a DSP, or dedicated hardware, for example, performs numerical calculations and information processing based on various programs, and controls hardware units. The hardware units are connected to each other via an electrical communication path (wire), such as a bus, for unidirectional or bidirectional communication. The controller 10b is configured to perform various processing as will be described later, which can be implemented by a logic circuit or by executing software. The processing executable by the controller 10b include various general image processing. The controller 10b can be obtained by combining hardware and software.

First, a configuration of the controller 10b will be described, and then a skin surface analysis method by the controller 10b will be described with reference to a specific example image.

### (Configuration of Controller 10b)

The controller 10b can take in an image from the outside directly or via the communicator 10a. The image taken in can be stored in the storage 10c. The image to be taken in is an image obtained by imaging the transcription surface of the transcription material 100 magnified by the stereo microscope 101, and serves as a basis for FIGS. 5Aand 5B, for example. The image to be taken in may be a color image or a grayscale image converted from a color image.

The controller 10b includes an image input section 20 to which a color image or a grayscale image is input. An image converted to grayscale by a grayscale processor 21, which will be described later, may be input to the image input section 20, or an image converted to grayscale in advance outside the skin surface analysis device 1 may be input to the image input section 20. Similarly to the reading of an image into the grayscale processor 21 described above, an image can be input to the image input section 20 by a user of the skin surface analysis device 1. A color image can be input to the image input section 20.

The controller 10b includes the grayscale processor 21 for converting, if an image taken in is a color image, the color image to grayscale. The color image does not have to be converted to grayscale and may be, as it is, subjected to the local image enhancement processing and subsequent processing, which will be described later.

For example, an image can be taken in by a user of the skin surface analysis device 1. For example, an image magnified by the stereo microscope 101 is captured by an imaging device (not shown) and the thus obtained image data can be read into the grayscale processor 21. In this example, an image of image data output from the imaging device and saved in the JPEG or the PNG format is used. However, the format is not limited thereto. Image data compressed in another compression format or a RAW image may also be used. In this example, an image is in a size of 1600 × 1200 pixels, but may be in any size.

The grayscale processor 21 converts a color image to grayscale with 8-bit depths, for example. Specifically, the grayscale processor 21 converts an image to an image of pixels whose sample value contains no information other than the luminance. This grayscale is different from a binary image, and expresses an image in colors from white of the strongest luminance to black of the weakest luminance, including gray shades. The depths are not limited to 8 bits, but can be any suitable values.

The controller 10b includes a local image enhancement processor 22. The local image enhancement processor 22 executes local image enhancement processing of enhancing the contrast of a local region of a grayscale image, which has been input to the image input section 20, to generate an enhanced image. This improves the visibility of the details of the image. Examples of the local image enhancement processing include processing, such as histogram equalization, of enhancing the contrast of a local region of an image to improve the visibility of the details.

The controller 10b includes a patch image generator 23. The patch image generator 23 is a section that divides the enhanced image generated by the local image enhancement processor 22 into a plurality of patch images. Specifically, the patch image generator 23 divides an enhanced image in a size of 1600 × 1200 pixels, for example, into images (i.e., patch images) each in a size of 256 × 256 pixels. The patch image generator 23 can also generate the patch images so that adjacent patch images partially overlap each other. That is, a patch image generated by the patch image generator 23 partially overlaps the adjacent patch images. The overlapping range can be set to about 64 pixels, for example. This set overlapping range can be referred to as a "64-pixel stride," for example. The pixel values described above are mere examples and may be any suitable values.

If an image is divided into a plurality of patch images without overlapping adjacent patch images, an edge of a skin ridge or a sweat droplet may happen to overlap the boundary between the adjacent patch images, which may degrade the accuracy in distinguishing the skin ridge or the sweat droplet overlapping the boundary by the machine learning identifier 24, which will be described later. By contrast, this example allows a skin ridge or a sweat droplet to be accurately distinguished even at the position described above, since adjacent patch images partially overlap each other.

The controller 10b includes the machine learning identifier 24. The machine learning identifier 24 is a section that receives the patch images generated by the patch image generator 23 and executes segmentation of each of the input patch images. The machine learning identifier 24 itself segments each input image by a known deep learning technique. Based on this segmentation, the machine learning identifier 24 determines, for example, to which category each pixel belongs and outputs the result as an output image. The machine learning identifier 24 includes an input layer to which an input image is input, an output layer that outputs an output image, and a plurality of hidden layers between the input and output layers. The machine learning identifier 24 learns a large quantity of teacher data to enable automatic extraction of a common feature and flexible determination. The learning has been completed.

In this example, the input and output images of the machine learning identifier 24 have the same resolution. In a case of a typical machine learning identifier, an input image has a higher resolution, and an output image is output at a lower resolution. In this example, however, the resolution of the output image is not reduced because the shape of fine skin ridges, the sizes of sweat droplets, and other factors need to be distinguished accurately. For example, if a patch image in a size of 256 × 256 pixels is input to the input layer of the machine learning identifier 24, an output image in a size of 256 × 256 pixels is output from the output layer.

The machine learning identifier 24 in this example can execute the detection of skin ridges and skin folds and the detection of sweat droplets at the same time. Specifically, the machine learning identifier 24 includes a skin ridge and skin fold detector 24a that detects skin ridges and skin folds, and a sweat droplet detector 24b that detects sweat droplets. Each of the skin ridge and skin fold detector 24a and the sweat droplet detector 24b can be constructed using, for example, Unet as a network.

The controller 10b includes a whole image generator 25. The whole image generator 25 is a section that generates a whole image by combining the patch images segmented and output from the machine learning identifier 24. Specifically, the whole image generator 25 combines the patch images output from the skin ridge and skin fold detector 24a into an image like the image before the division to generate a whole image for distinguishing skin ridges and skin folds, and combines the patch images output from the sweat droplet detector 24b in the same manner to generate a whole image for distinguishing sweat droplets. The whole image is in the same size as the image before the division.

The controller 10b includes a likelihood map generator 26. The likelihood map generator 26 is a section that generates a likelihood map image of skin ridges from the whole image for distinguishing skin ridges and skin folds generated by the whole image generator 25 based on a result of the segmentation by the machine learning identifier 24. The likelihood map image is an image color-coded according to the likelihoods of pixels and relatively shows which pixel has a higher likelihood or a lower likelihood. For example, a color map image of pixels of the highest likelihood shown in red, pixels of the lowest likelihood in blue, and pixels therebetween expressed in 8-bit depths can be used as a likelihood map image of skin ridges and skin folds. This display format is a mere example and may be grayscale or a display format with different lightness, and may have depths other than 8 bits.

The likelihood map generator 26 generates a likelihood map image of sweat droplets from the whole image for distinguishing sweat droplets generated by the whole image generator 25 based on a result of the segmentation by the machine learning identifier 24. A color map image of pixels of the highest likelihood of a sweat droplet shown in red, pixels of the lowest likelihood of a sweat droplet in blue, and pixels therebetween expressed in 8-bit depths can be used as a likelihood map image of sweat droplets. Similarly to the case of the skin ridges and skin folds, the likelihood map image of sweat droplets may be displayed in grayscale, a display format with different lightness, and may have depths other than 8 bits.

The controller 10b has a binarization processor 27. The binarization processor 27 is a section that executes binarization processing on the likelihood map image, which has been generated by the likelihood map generator 26, to generate a binary image (i.e., a black and white image). The threshold Th used in the binarization processing may be set to be any value. For example, Th can be set to 150 (Th = 150) in the case of 8-bit depths. It is possible to distinguish between skin folds and skin ridges by determining, for example, a black portion to be skin folds and a white portion to be skin ridges, using a likelihood map image based on a whole image for distinguishing skin ridges and skin folds. It is also possible to distinguish between sweat droplets and portions other than sweat droplets by determining, for example, white portions to be sweat droplets and black portions to be portions other than sweat droplets, based on a whole image for distinguishing sweat droplets.

The controller 10b includes a region extractor 28. The region extractor 28 is a section that extracts a skin ridge region based on a binary image generated by the binarization processor 27. Specifically, if white portions represent skin ridges in the binary image, a group of white pixels in the binary image is extracted as a skin ridge region. The region extractor 28 may extract a skin fold region based on a binary image generated by the binarization processor 27. In this case, if black portions represent skin folds in the binary image, a group of black pixels in the binary image is extracted as a skin fold region. The region extractor 28 may extract skin folds and thereafter extract the other region as the skin ridge region. Alternatively, the region extractor 28 may extract skin ridges and thereafter extract the other region as the skin fold region. As described below in the skin ridge analyzer 30, a grayscale image, in which a skin ridge is close to white, and a skin fold is close to black, can be used to observe the condition of the skin surface. In this case, the skin folds are represented by a luminance value close to black (0 for 8-bit images) and the skin ridges are represented by a luminance value close to white (255 for 8-bit images), allowing quantitative representation of the distribution and changes in the skin folds and skin ridges.

The controller 10b includes a sweat droplet extractor 29. The sweat droplet extractor 29 is a section that extracts sweat droplets based on a likelihood map image of sweat droplets. Specifically, if white (or red) portions in the likelihood map image of sweat droplets represent sweat droplets, a group of white (or red) pixels in the likelihood map image of sweat droplets is extracted as sweat droplets. The sweat droplet extractor 29 may extract portions other than sweat droplets, based on a likelihood map image of sweat droplets. In this case, if black (or blue) portions in the likelihood map image of sweat droplets represent other portions than sweat droplets, a group of black (or red) pixels in the likelihood map image of sweat droplets is extracted as other portions than sweat droplets. The sweat droplet extractor 29 may extract portions other than sweat droplets from the likelihood map image of sweat droplets and thereafter extract other portions as sweat droplets.

The transcription material 100 may contain bubbles, which may be erroneously distinguished as sweat droplets. In this case, a distinguishing method using dimensions is also applied. For example, a threshold for distinguishing is set to "40 µm" as an example. A small region with a diameter of 40 µm or less is distinguished as a bubble, and only a region with a diameter over 40 µm is distinguished as a sweat droplet. Another example of the threshold for distinguishing is an area. For example, the area of a circle with a diameter of 40 µm is obtained in advance. A small region with an area equal to or smaller than that area is distinguished as a bubble, and only a region with an area greater than that area is distinguished as a sweat droplet. The "diameter" may be, for example, a longitudinal diameter in a case of an elliptic approximation.

The controller 10b includes a skin ridge analyzer 30. The skin ridge analyzer 30 is a section that calculates the area of a skin ridge region extracted by the region extractor 28. The skin ridge analyzer 30 can grasp the shape of a skin ridge by, for example, generating an outline surrounding a skin ridge region extracted by the region extractor 28. The skin ridge analyzer 30 can calculate the area of the skin ridges by obtaining the area of the region surrounded by the outline of the skin ridge. The skin ridge analyzer 30 can also grasp the shape of a skin fold by generating, for example, an outline surrounding a skin fold region extracted by the region extractor 28. The skin ridge analyzer 30 can also calculate the area of the skin folds by obtaining the area of the region surrounded by the outline of the skin fold.

The skin ridge analyzer 30 sets a plurality of grids in a predetermined size on a binary image or a grayscale image, and calculates the ratio between the skin ridge region and the skin fold region in each grid. Specifically, as an example, assume that a grid is set to divide a binary image into nine equal images, namely, first to ninth divisional images. In this case, the skin ridge analyzer 30 calculates the areas of the skin ridge region and the skin fold region included in each divisional image to obtain the ratio between the areas of the skin ridge region and the skin fold region. If, for example, the fineness of a skin surface needs to be evaluated, the fineness of the skin surface can be evaluated based on the ratio between the skin ridge region and the skin fold region in the grid set on a binary image or a grayscale image. A ratio of the skin ridge region higher than or equal to a predetermined value can be a criterion for determining a coarse skin. On the other hand, a ratio of the skin ridge region lower than the predetermined value can be a criterion for determining a fine skin.

Used in the following description of the embodiment is a result of analysis of skin ridges and skin folds (in which a skin ridge is close to white, and a skin fold is close to black) on a grayscale image using the skin ridge analyzer 30. A healthy person has a skin surface with a clear boundary between a skin ridge and a skin fold, which allows measurement of the area of the skin ridge. On the other hand, an atopic dermatitis patient may have a skin surface with an unclear boundary between a skin ridge and a skin fold. In this case, a grayscale image is used as it is for analysis; the ratios between the skin ridge and the skin fold in a plurality of grid are obtained; and grayscale values of the pixels in the grids are used to analyze the ratios between the skin ridge and the skin fold, and the analysis result is displayed in a histogram, which can be used as criteria for determining the fineness or other characteristics of the skin (which will be described later).

The skin ridge analyzer 30 converts the ratio between the skin ridge region and the skin fold region in each grid into numbers to calculate a frequency distribution. Specifically, the skin ridge analyzer 30 calculates the ratios between the areas of the skin ridge region and the skin fold region, and then converts the ratios into numbers and summarizes the data in the form of a frequency distribution table. In addition, a skin ridge analyzer 30 can calculate the center of gravity of each skin ridge region, and a perimeter length, rectangular approximation, elliptic approximation, circularity, aspect ratio, density, and other characteristics of the skin ridge region.

In some state of a disease, there may be a groove formed in a part of a skin ridge. In this case, an unraised portion, that is, a recess is present in the skin ridge region extracted. Dividing the skin ridge region by this recess can serve as a criterion in determining the state of the disease and making a clinical evaluation. To make this happen, the skin ridge analyzer 30 determines, after extracting the skin ridge region, whether each portion of the extracted skin ridge region is raised and divides the skin ridge region by a portion determined to be unraised. For example, a skin ridge region may include a groove-like portion. In this case, the skin ridge region is not fully raised but partially (i.e., the groove-like portion is) recessed. The portion determined to be unraised, that is, the portion determined to be a recess is the groove-like portion which divides a single skin ridge region into a plurality of skin ridge regions.

The controller 10b includes the sweat droplet analyzer 31. The sweat droplet analyzer 31 calculates a distribution of the sweat droplets extracted by the sweat droplet extractor 29. The sweat droplet analyzer 31 can calculate, for example, the number of sweat droplets per unit area (e.g., 1 mm² or 1 cm²) of a skin surface, the size (i.e., the diameter) of each sweat droplet, the area of the sweat droplet, and other factors. The sweat droplet analyzer 31 can also calculate the total area of the sweat droplets per unit area of a skin surface.

The controller 10b includes an information output section 32. The information output section 32 generates and outputs information on the shape of a skin ridge region extracted by the region extractor 28 and information on sweat droplets extracted by the sweat droplet extractor 29. The information on the shape of a skin ridge region includes results of calculation by the skin ridge analyzer 30. Examples may include the area of a skin ridge region, the center of gravity the skin ridge region, and a perimeter length, rectangular approximation, elliptic approximation, circularity, aspect ratio, density, and other characteristics of the skin ridge region. On the other hand, the information on sweat droplets includes results of calculation by the sweat droplet analyzer 31. Examples may include the number of sweat droplets per unit area, the total area of the sweat droplets per unit area, and other characteristics.

### (Skin Surface Analysis Method)

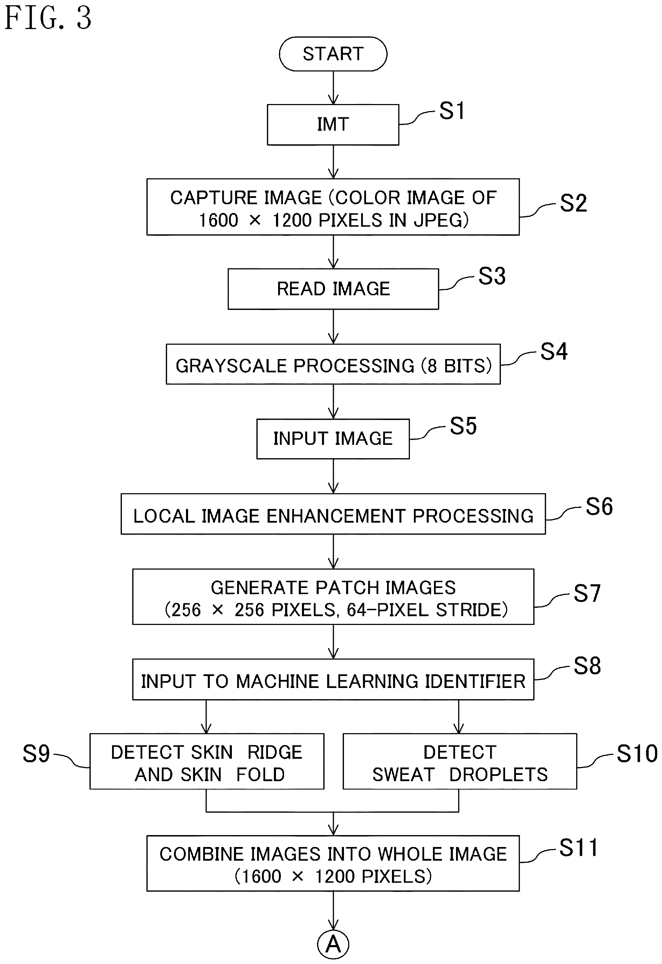

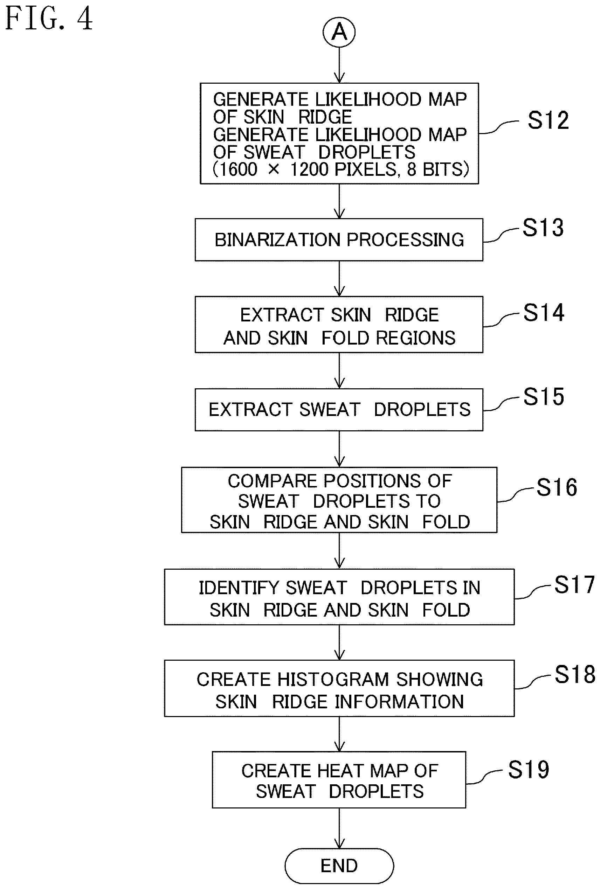

Next, a skin surface analysis method using the skin surface analysis device 1 configured as described above will be described with reference to specific example images. The flow of the skin surface analysis method is as shown in the flowcharts of FIGS. 3 and 4. In step S1 of the flowchart shown in FIG. 3, IMT is performed. In this step, as shown in FIG. 1, a dental silicone impression material is applied like a film onto a skin surface and left for a predetermined time, and then peeled off from the skin to obtain the transcription material 100 to which a human skin surface microstructure is transcribed.

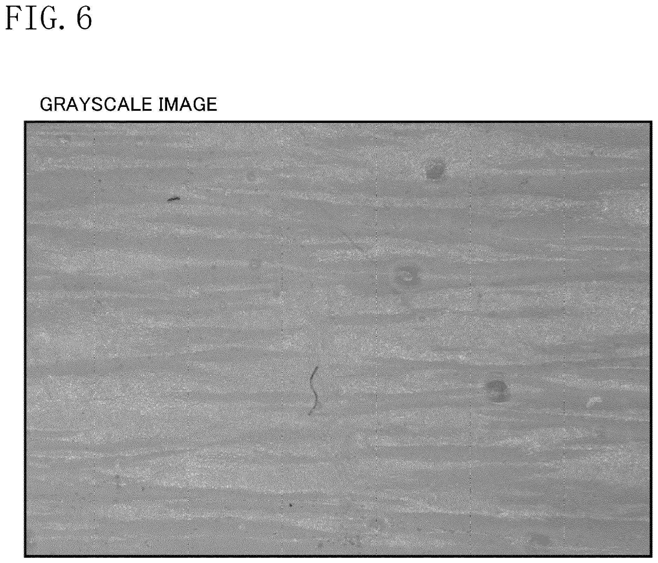

The process then proceeds to step S2. In step S2, the transcription material 100 is set in the stereo microscope 101 and observed at a predetermined magnification, and the observed field of view is imaged by an imaging device. In this manner, a color image (1600 × 1200 pixels) is obtained in the JPEG or the PNG format. Subsequently, the process proceeds to step S3, in which the color image captured by the imaging device is read into the controller 10b of the skin surface analysis device 1. The process then proceeds to step S4, in which the grayscale processor 21 (shown in FIG. 2) converts the color image read in step S3 to grayscale with 8-bit depths to generate a grayscale image. An example of the generated grayscale image is shown in FIG. 6. A light-color portion is a skin ridge and a dark-color portion is a skin fold on the grayscale image, but the boundary therebetween is unclear. It takes thus time for an inspector, during distinguishing, to determine where in the image is a skin fold or a skin ridge, and a limited number of samples can be processed within a certain time. If the image read into the controller 10b is a grayscale image, no grayscale processing is necessary.

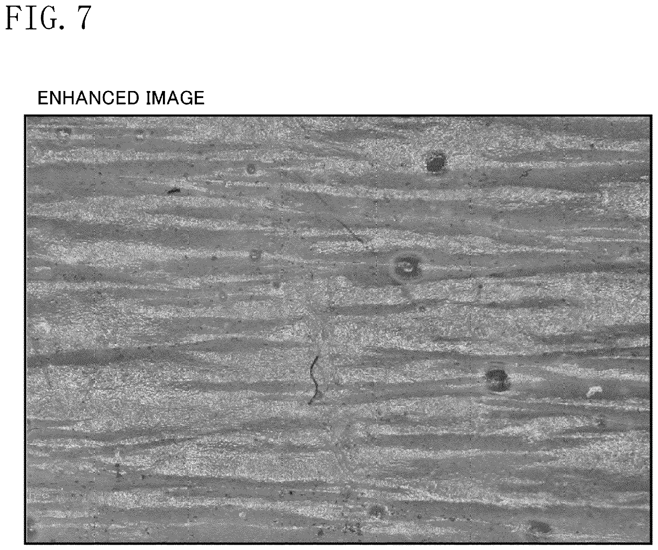

In the following step S5, the grayscale image is input to the image input section 20. This step corresponds to "image input." Then, in step S6, the local image enhancement processor 22 executes local image enhancement processing on the grayscale image that is input in step S5. This step corresponds to "local image enhancement." FIG. 7 shows an image subjected to the local image enhancement processing. It can be seen that the image shown in FIG. 7 exhibits a more enhanced contrast of a local region and a more improved visibility of the details than the image shown in FIG. 6.

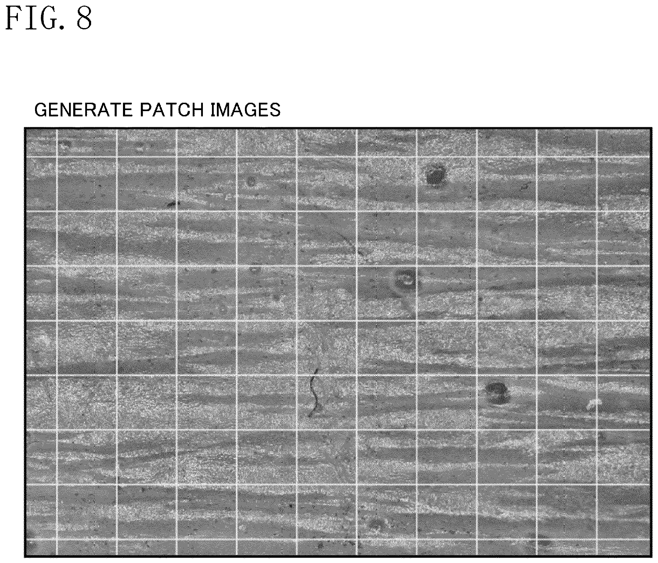

The process then proceeds to step S7. In step S7, the patch image generator 23 divides the enhanced image generated in step S6 into a plurality of patch images. FIG. 8 shows the division into patch images, and grid lines correspond to the boundaries of the patch images. In this figure, the patch images adjacent to each other in the vertical and horizontal directions of the figure overlap each other at a "64-pixel stride." This step corresponds to "patch image generation."

After generating the patch images, the process proceeds to step S8. In step S8, the patch images generated in step S7 are input to the machine learning identifier 24 which executes segmentation of the input patch images. At this time, the same patch images are input to both the skin ridge and skin fold detector 24a and the sweat droplet detector 24b (steps S9 and S10). This step corresponds to "segmentation."

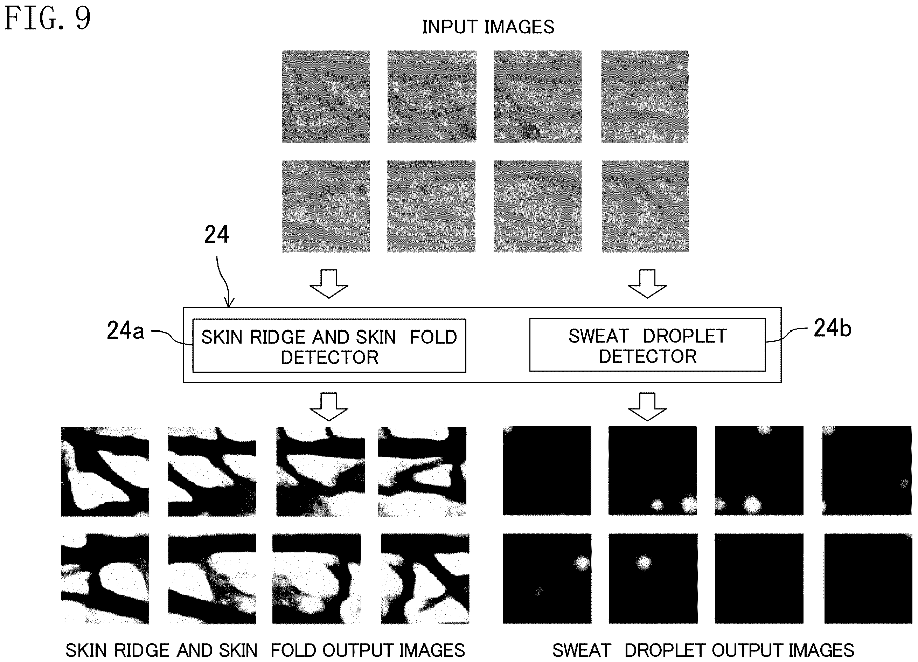

Specifically, as shown in FIG. 9, if there are eight patch images present as input images, the eight patch images are input to the skin ridge and skin fold detector 24a and to the sweat droplet detector 24b, as well. The skin ridge and skin fold detector 24a generates and outputs an output image in which the color of each pixel is set to be whiter with a higher likelihood of skin ridges and blacker with a lower likelihood of skin ridges (i.e., with an increasing likelihood of skin folds) for all the input images. The sweat droplet detector 24b generates and outputs an output image in which the color of each pixel is set to be whiter with a higher likelihood of a sweat droplet and blacker with a lower likelihood of a sweat droplet for all the input images.

FIG. 9 shows example skin ridge and skin fold output images that are output from the skin ridge and skin fold detector 24a, and example sweat droplet output images that are output from the sweat droplet detector 24b. In the skin ridge and skin fold output images, the white portions correspond to skin ridge regions, and the black portions correspond to skin fold regions. In the sweat droplet output image, white portions correspond to sweat droplets.

In this example, as described above, in dividing the enhanced image into a plurality of patch images in step S7, adjacent patch images are overlapped with each other. If the adjacent patch images do not overlap each other, an edge of a skin ridge or a sweat droplet may happen to overlap the boundary between the adjacent patch images, which may degrade the accuracy in distinguishing the skin ridge or the sweat droplet overlapping the boundary. By contrast, in this example, the adjacent patch images partially overlap each other, allowing a skin ridge or a sweat droplet to be accurately distinguished even at the position described above.

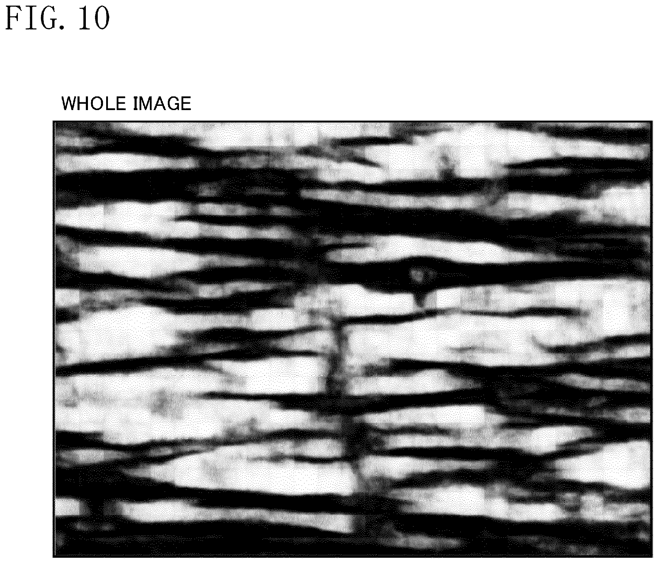

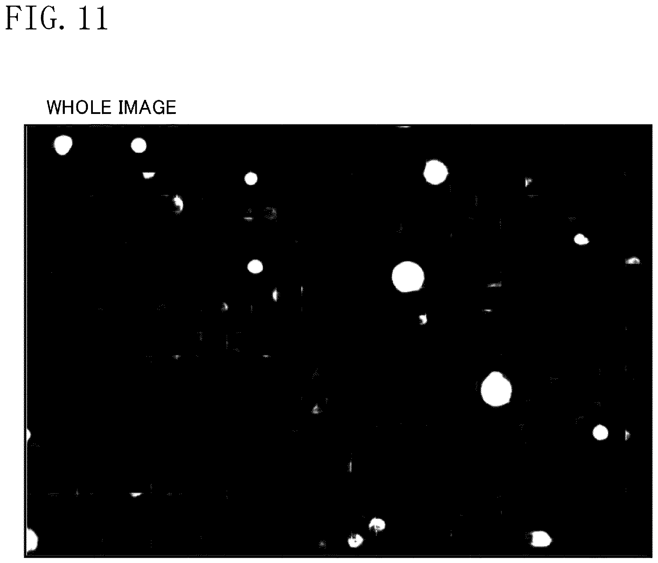

After that, the process proceeds to step S11, in which the skin ridge and skin fold output images (i.e., patch images) after step S9 are combined to generate a whole image as shown in FIG. 10. Further, in step S11, the sweat droplet output images (i.e., patch images) after step S9 are combined to generate a whole image as shown in FIG. 11. Each whole image includes the same number of pixels as the image input in step S5. This step corresponds to "whole image generation."

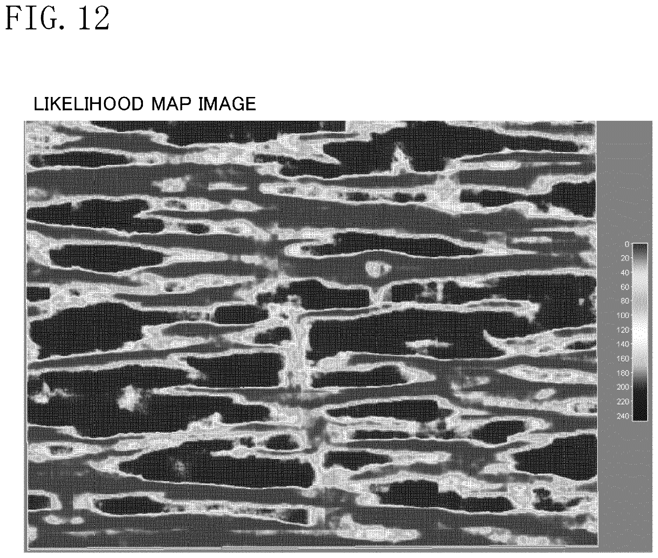

Subsequently, the process proceeds to step S12 shown in FIG. 4, in which the likelihood map generator 26 generates, from the whole image generated in step S11, a likelihood map image of skin ridges and a likelihood map image of sweat droplets based on a result of the segmentation. This step corresponds to "likelihood map generation." FIG. 12 shows an example likelihood map image of skin ridges. In this figure, a grayscale image is shown for the sake of simplicity. However, in this example, a color image of pixels of the highest likelihood of skin ridges shown in red, pixels of the lowest likelihood of skin ridges in blue, and pixels therebetween expressed in 8-bit depths is used. This facilitates distinguishing between a skin ridge region and a skin fold region.

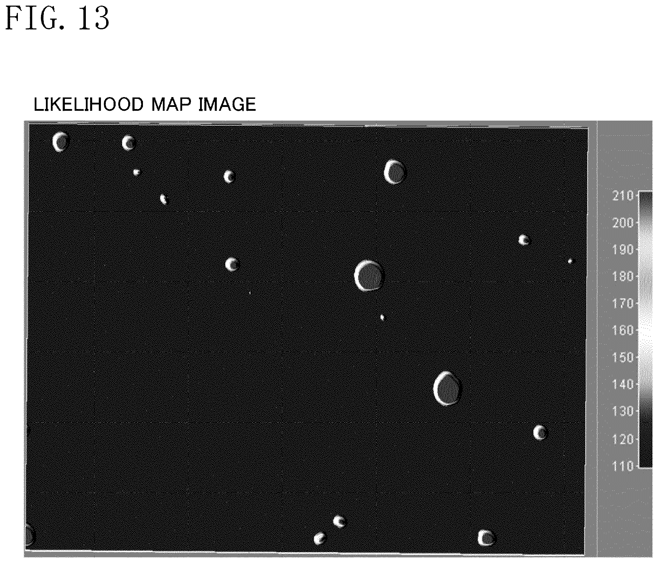

FIG. 13 shows an example likelihood map image of sweat droplets. This image is also a color image in the example, with pixels of the highest likelihood of sweat droplets shown in red, pixels of the lowest likelihood of sweat droplets in blue, and pixels therebetween expressed in 8-bit depths. This facilitates distinguishing sweat droplets.

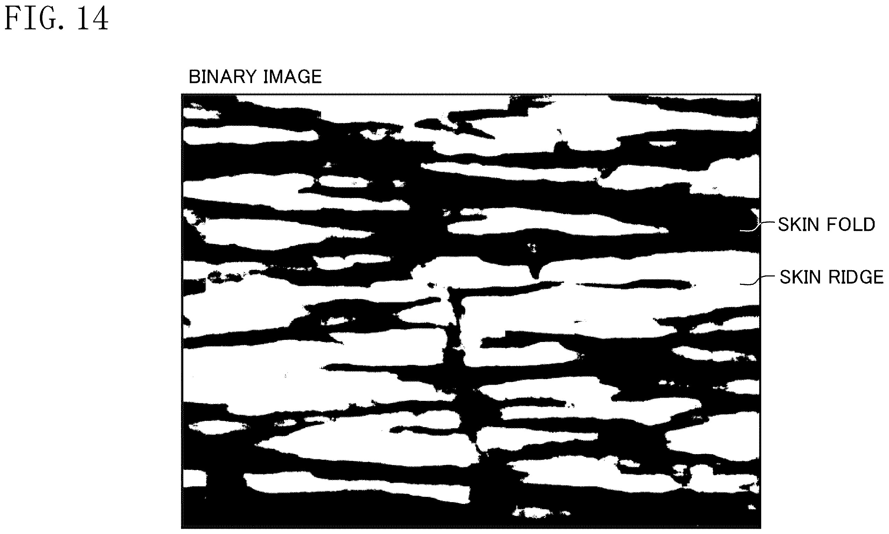

After generating the likelihood map image of skin ridges and the likelihood map image of sweat droplets, the process proceeds to step S13. In step S13, binarization processing is executed on the likelihood map image of skin ridges, which has been generated in step S12, to generate a binary image. This step is executed by the binarization processor 27 and corresponds to the "binarization processing". FIG. 14 shows the binary image generated by executing the binarization processing on the likelihood map image of skin ridges.

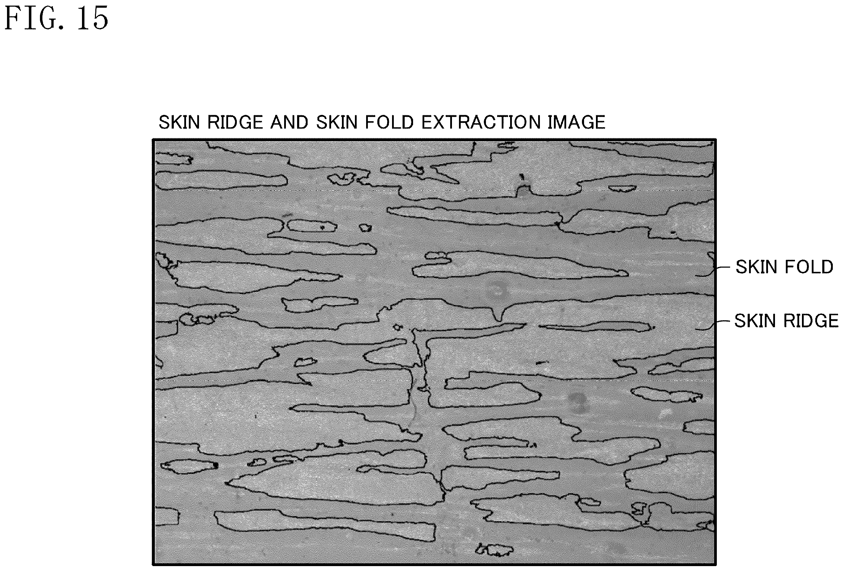

After that, the process proceeds to step S14, in which the region extractor 28 extracts a skin ridge region based on the binary image generated in step S13. At this time, a skin fold region may be extracted. FIG. 15 shows an image where skin ridges and skin folds are extracted, and skin ridge regions are surrounded by black lines. This step corresponds to "region extraction."

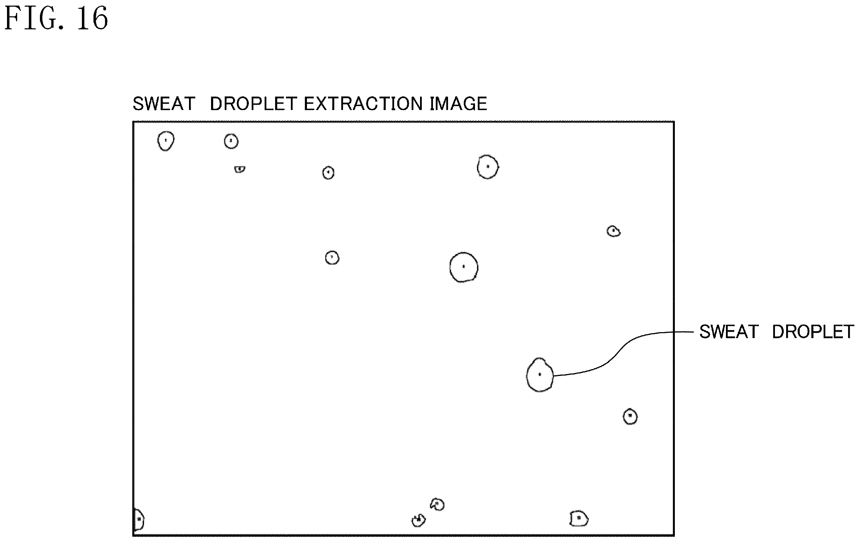

The process proceeds to step S15, in which the sweat droplet extractor 29 extracts sweat droplets based on the likelihood map image of sweat droplets generated in step S12. This step corresponds to the "sweat droplet extraction." FIG. 16 shows an image where sweat droplets are extracted, and sweat droplets are surrounded by black lines.

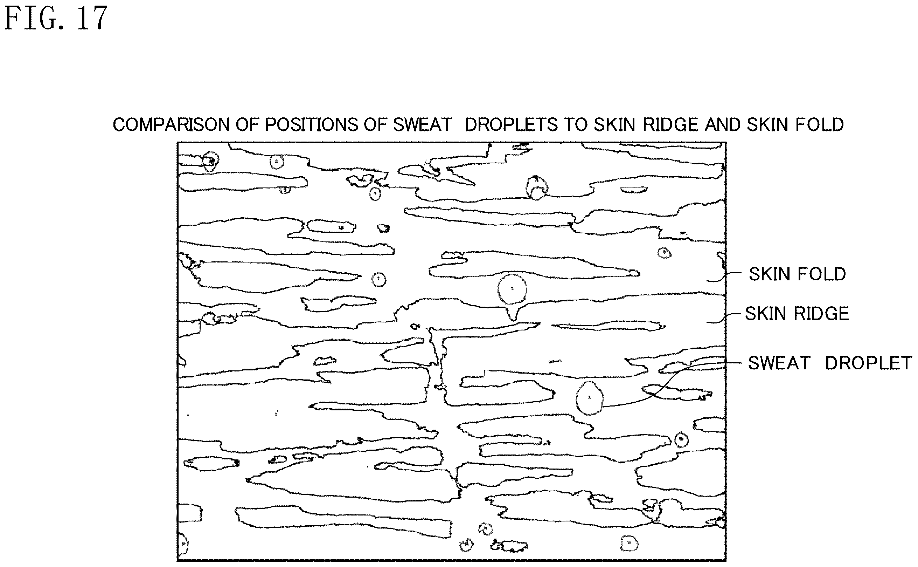

The process then proceeds to step S16. In step S16, comparison is made between the positions of the sweat droplets and the skin ridges and skin folds. The positions and ranges of the sweat droplets can be specified by XY coordinates on the image. The positions and ranges of skin ridges and skin folds can also be specified by the XY coordinates on the image. The image for specifying the positions and ranges of sweat droplets and the image for specifying the positions and ranges of skin ridges and skin folds are originally the same; thus, the sweat droplets can be placed on the image showing skin ridges and skin folds as shown in FIG. 17. The relative positional relationship between the sweat droplets and the skin ridges and skin folds can be grasped in this manner. At this time, the region of the skin ridges and the coordinate of the center of gravity of the sweat droplets can be used.

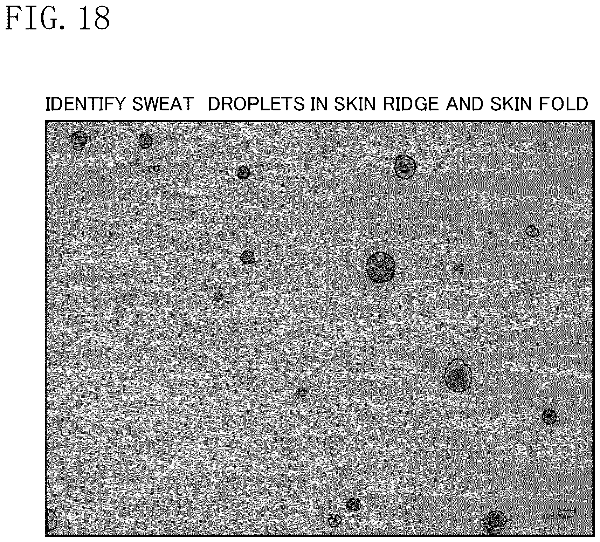

The process then proceeds to step S17. In step S17, the sweat droplets in skin ridges and skin folds are identified. FIG. 18 shows an image in which sweat droplets in skin ridges and skin folds are identified. This image allows distinguishing between sweat droplets in skin ridges and sweat droplets in skin folds. In FIG. 18, figures in the shape close to circle correspond to sweat droplets.

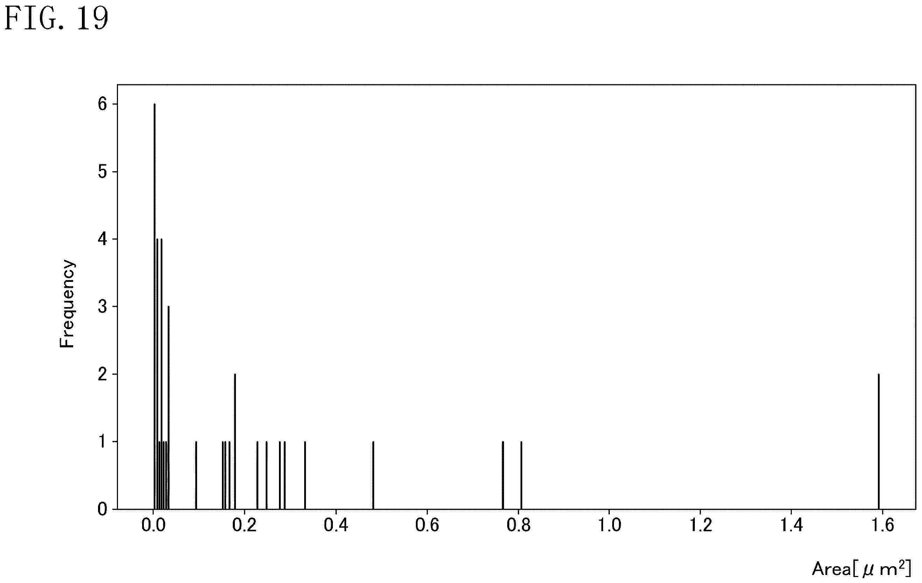

After the identification, the process proceeds to step S18 and step S19. Either step S18 or S19 may be performed first. In step S18, a histogram showing skin ridge information is created and displayed on the monitor 11. First, the skin ridge analyzer 30 calculates the respective areas of the skin ridge regions extracted in step S14. Then, as shown in FIG. 19, a histogram is created in which the horizontal axis represents the areas and the vertical axis represents the frequency. This step corresponds to "skin ridge analysis." This allows grasping of a distribution of the areas of the skin ridge regions. For example, an atopic dermatitis tends to cause an increased area of a single skin ridge. Thus, high frequencies of large areas indicate the strong tendency of a sweating disturbance due to atopic dermatitis.

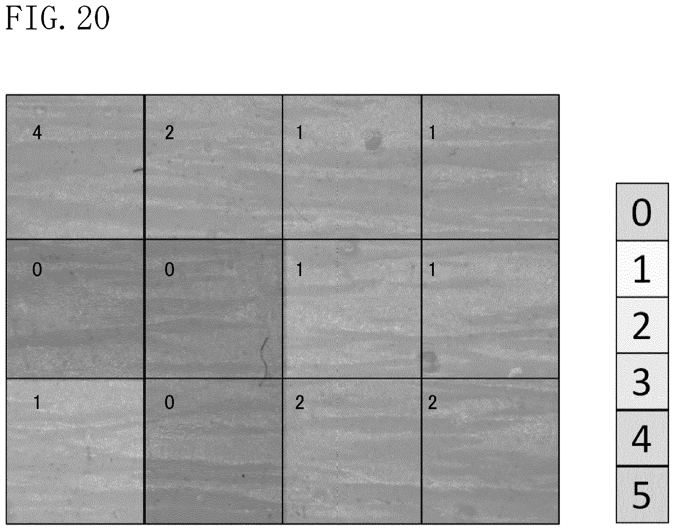

In step S19, a heat map image of sweat droplets is created and displayed on the monitor 11. First, the sweat droplet analyzer 31 calculates a distribution of sweat droplets extracted in step S15. For example, as shown in FIG. 20, grids are formed on an image obtained by imaging the transcription material 100, and the number of sweat droplets in each grid is counted. This can be made by determining in which grid the coordinates of the center of gravity of a sweat droplet extracted in step S15 are included. For example, a grid with no sweat droplet, a grid with one sweat droplet, a grid with two sweat droplets, a grid with three sweat droplets, ... , are color-coded to color the respective grids, thereby making it possible to grasp the distribution of the sweat droplets. Such a color-coded image can be called a "heat map image." This step corresponds to the "sweat droplet analysis." A sparse distribution of sweat droplets indicates the strong tendency of a sweating disturbance due to atopic dermatitis.

The creation of a heat map image is also advantageous in determining, as a pattern, the sweating and conditions of skin ridges in a small area, which cannot be obtained from individual analysis areas or cannot be determined even from a wide area if the entire area is averaged. Heat map images may be arranged in time series and displayed on the monitor 11. For example, heat map images are generated when one week, two weeks, and three weeks have elapsed since the start of treatment of an atopic dermatitis patient, and are displayed in the form of a list, thereby making it possible to determine whether the symptom improves and make quantitative determination on the progress.

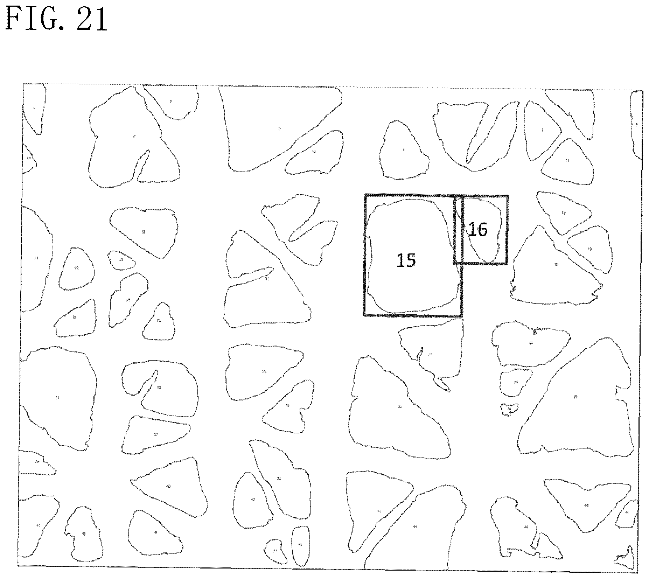

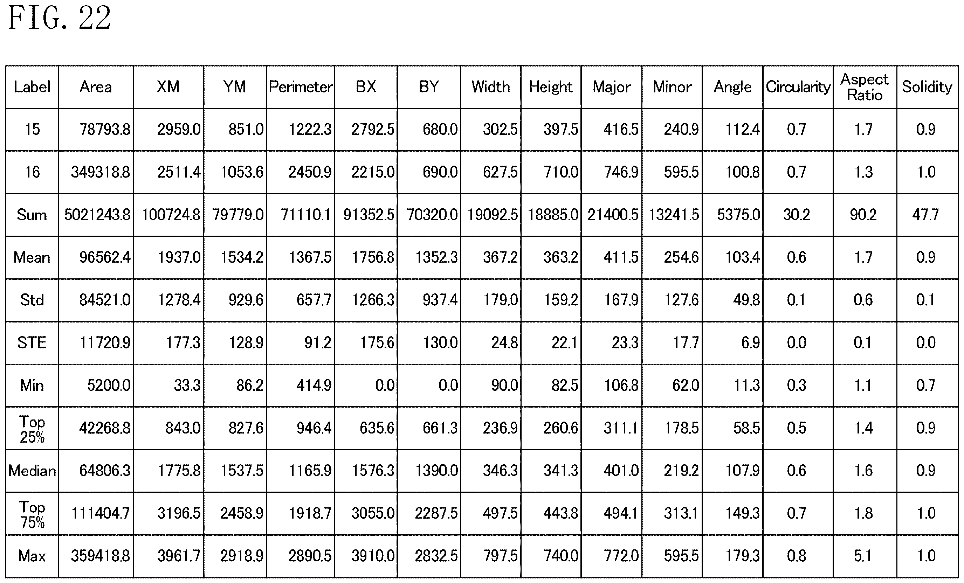

FIG. 21 shows an example skin ridge region image in which lines surrounding respective skin ridge regions extracted in step S14 are shown. The image shown in this figure is generated by the skin ridge analyzer 30 and can be displayed on the monitor 11. If a fifteenth skin ridge region indicated by "15" and a sixteenth skin ridge region indicated by "16" are present in the figure, the skin ridge analyzer 30 creates a table showing results of measurement of specifications as shown in FIG. 22 and displays the table on the monitor 11.

In the table shown in FIG. 22, "Label" is provided to distinguish between the fifteenth skin ridge region and the sixteenth skin ridge region. The specifications include "Area" indicating the area of the skin ridge region, "XM" and "YM" indicating the center of gravity of the skin ridge region, "Perimeter" that is a perimeter length of the skin ridge region, "BX," "BY," "Width" and "Height" indicating the rectangular approximation, "Major," "Minor" and "Angle" indicating the elliptic approximation, "Circularity," "Aspect Ratio," and "Solidity" indicating the density. The skin ridge analyzer 30 can calculate these specification values, using image analysis software, for example. With the use of not only one index but a plurality of indices in this manner, determination can be made in association with clinical information. These indices, too, can contribute to distinguishing the fineness of a skin surface. It is thus possible to distinguish the fineness of a skin surface using the machine learning identifier 24. Further, as shown in FIG. 22, statistical processing (e.g., sum, maximum, minimum, or deviation) is also possible.

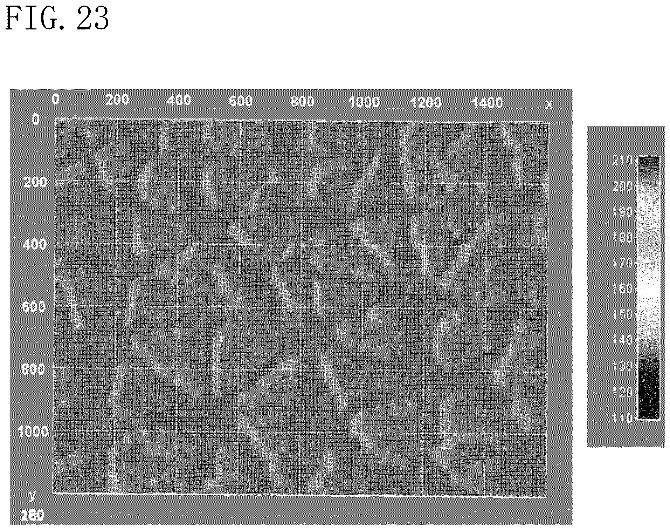

FIG. 23 is a graph showing a two-dimensional distribution of skin ridges and skin folds per grid of 128 × 128 pixels. Such a graph can be generated by the skin ridge analyzer 30 and displayed on the monitor 11. For example, the graph can be displayed as an 8-bit color image in which a skin ridge region is shown in red and a skin fold region in blue. For example, the graph may be used as an example method for expressing the fineness of a skin surface or an improvement in symptoms. The way of expressing can be a heat map, or can be a histogram of numbers converted from the ratios between the areas of the skin ridges and skin folds. According to this histogram, the frequency is high around a median value in the case of a fine skin surface, whereas the distribution is wide over the range and spreads toward ends in the case of an atopic dermatitis. In this manner, two-dimensional information can be quantified, and used as diagnostic information.

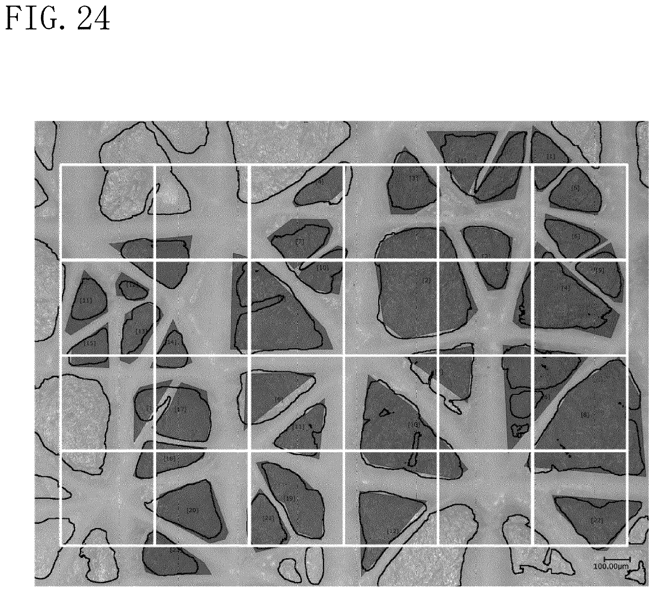

FIG. 24 shows a case in which the skin ridge analyzer 30 sets a plurality of (twenty-four in this example) grids in a predetermined size on an image and calculates the ratio between the skin ridge region and the skin fold region in each grid. In this case, the ratio between the skin ridge region and the skin fold region in each grid is converted into numbers, thereby making it possible to calculate the frequency distribution and display the distribution in the form of a histogram on the monitor 11. For example, in order to evaluate the fineness of the skin surface, a method using only the area of the skin ridges is conceivable. In this case, however, if two adjacent skin ridges are extremely close to each other and distinguished as one skin ridge, the area is determined to be about twice the actual size, which may result in an inaccurate analysis result. The obtainment of the ratio between skin ridges and skin folds in each grid as in this example allows quantitative calculation of the fineness of a skin surface.

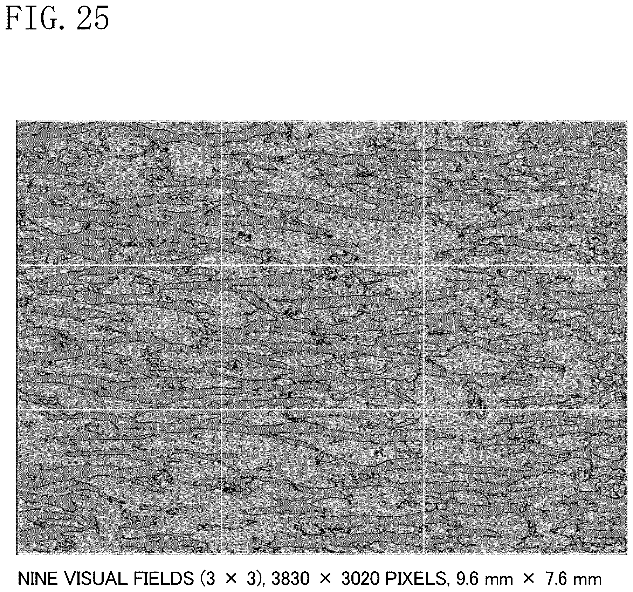

FIG. 25 shows an image obtained by combining imaging regions of nine (3 × 3 = 9) fields of view. This image enables observation of a wide area. Of this wide area, an image of a field of view with average sweating is subjected to the various analyses described above. For example, if focus is placed on only one field of view, it is impossible to distinguish among a field of view with a small amount of sweat, a field of view of a large amount of sweat, or a field of view of an average amount of sweat. By analyzing sweat droplets in all fields of view in a wide area, i.e., about nine fields of view, it is possible to exclude a field of view with a small amount of sweat and a field of view with a large amount of sweat, and select a field of view with an average amount of sweat, that is, a field of view suitable for skin surface analysis. The analysis result is therefore accurate. In the case of analysis by an inspector, only about three fields of view are processed due to time constraints, whereas the present invention allows analysis of a large number of fields of view, well in excess of three, enabling more accurate analysis of a skin surface.

The skin ridge analyzer 30 can also arrange images, such as the image shown in FIG. 25, in time series and display the images on the monitor 11. For example, images are generated as shown in FIG. 25 when one week, two weeks, and three weeks have elapsed since the start of treatment of an atopic dermatitis patient, and are displayed in the form of a list on the monitor 11, thereby making it possible to determine whether the symptom improves and make quantitative determination on the progress.

### (Quantification of Fineness of Skin Based on Ratio between Skin Ridges and Skin Folds)

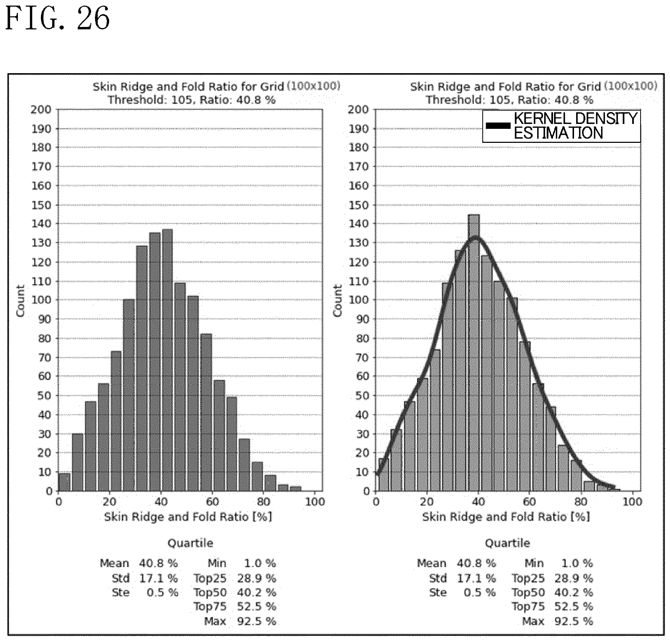

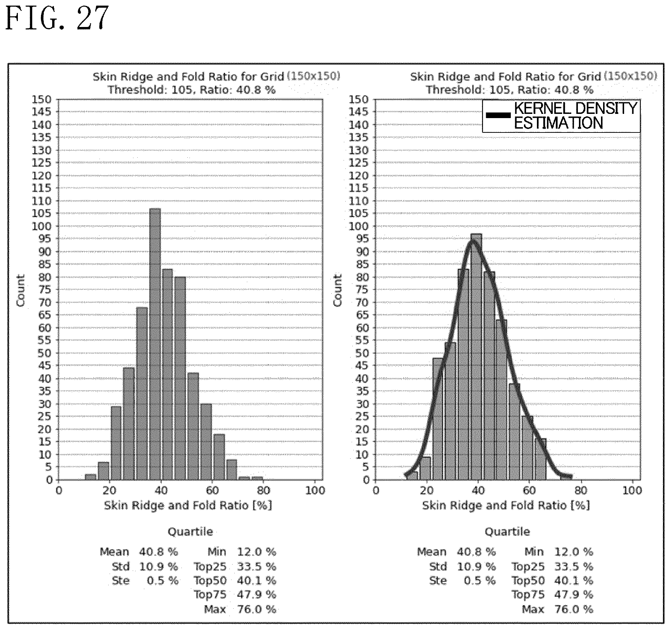

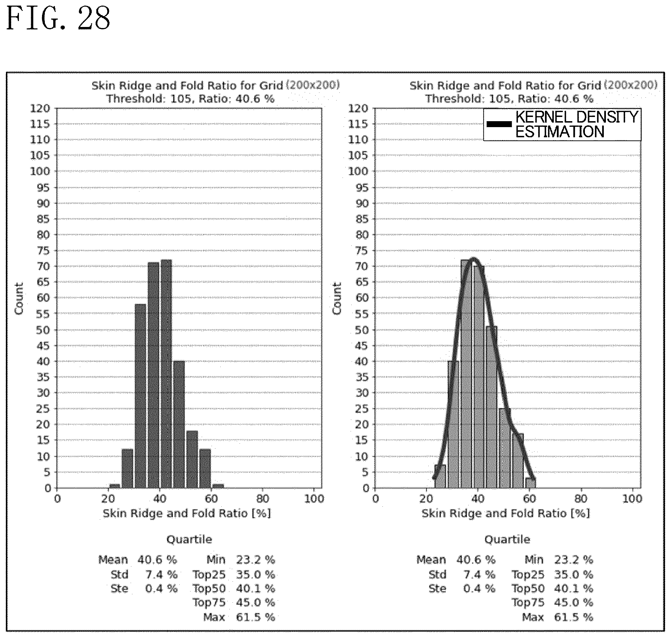

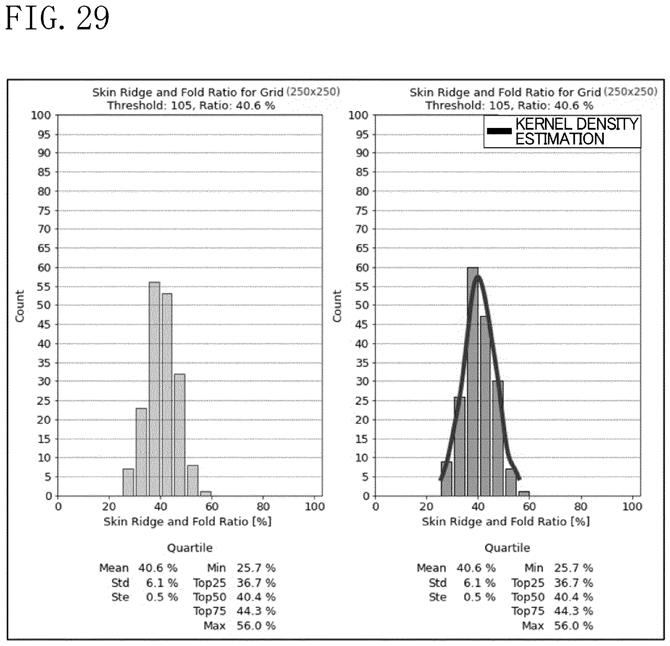

FIG. 26 is a graph (histogram) showing the ratio between skin ridges and skin folds on a forearm of a healthy person with a grid in a size of 100 × 100 pixels in a grayscale image. The horizontal axis represents the ratio between skin ridges and skin folds, while the vertical axis represents the count. The graph on the right of FIG. 26 also shows a graph of kernel density estimation. Similarly, FIG. 27 shows a case of a grid size of 150 × 150 pixels; FIG. 28 shows case of a grid size of 200 × 200 pixels; and FIG. 29 shows a case of a grid size of 250 × 250 pixels.

A fine skin, such as a skin of a forearm of a healthy person, has a distribution with a peak at a central portion in any grid in the pixel size of 100 × 100, 150 × 150, 200 × 200, or 250 × 250. In addition, since the ratio between skin ridges and skin folds is known, it is possible to quantify, based on the grid size, not only the size of the skin ridges but also the size of the skin folds.

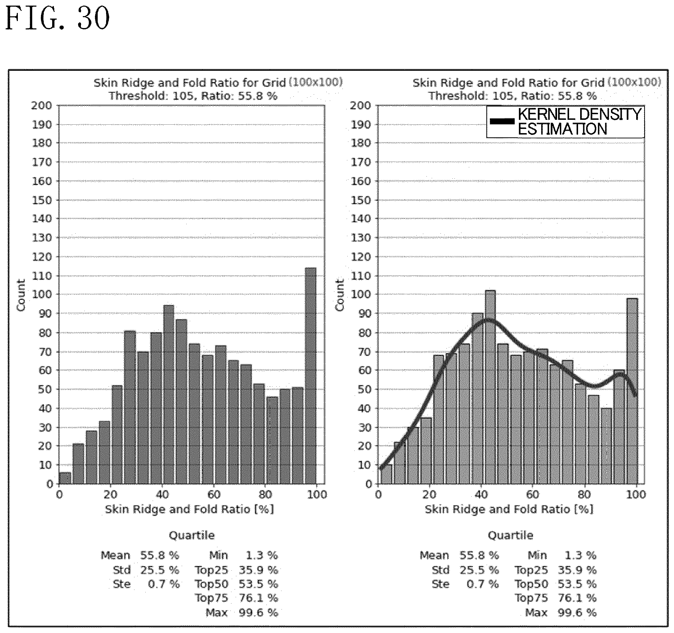

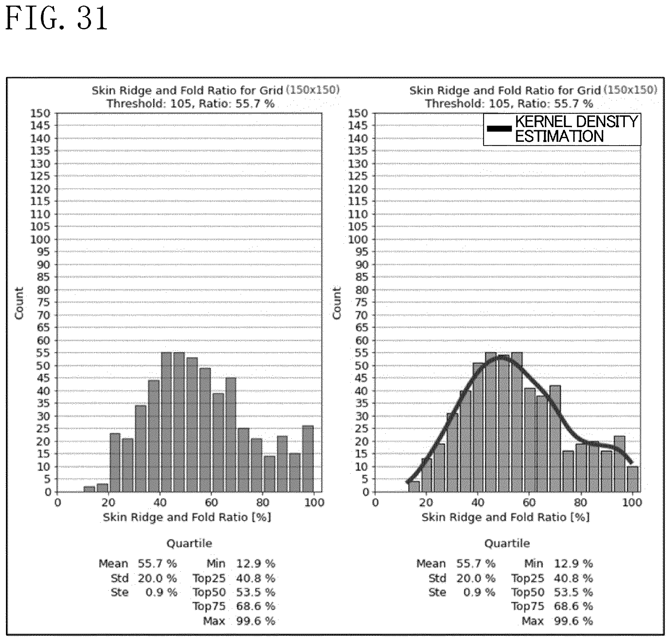

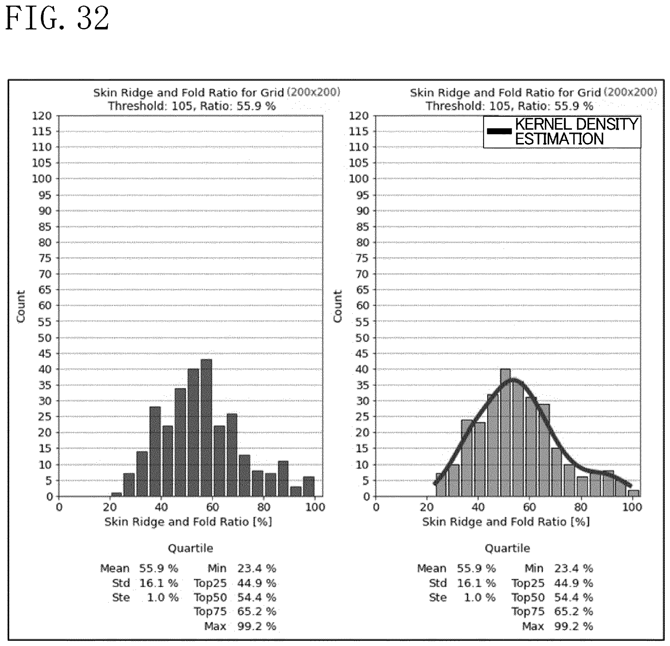

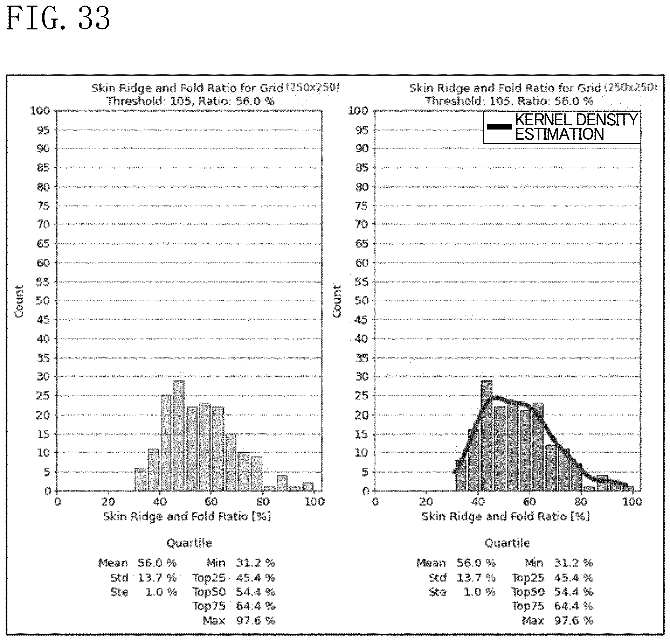

Next, the cases of an atopic dermatitis patient will be described. FIGS. 30 to 33 are graphs showing the ratios between skin ridges and skin folds on a thigh of an atopic dermatitis patient, and correspond to FIGS. 26 to 29, respectively. As compared to the graphs of FIGS. 26 to 29 showing the healthy person, a peak is shifted from the center or a plurality of peaks are found. By viewing these graphs, the difference between the healthy person and the atopic dermatitis patient can be grasped.

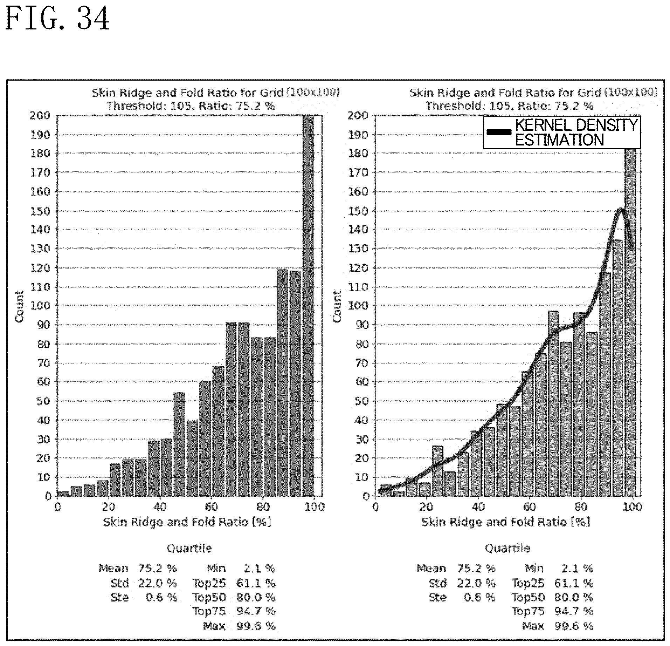

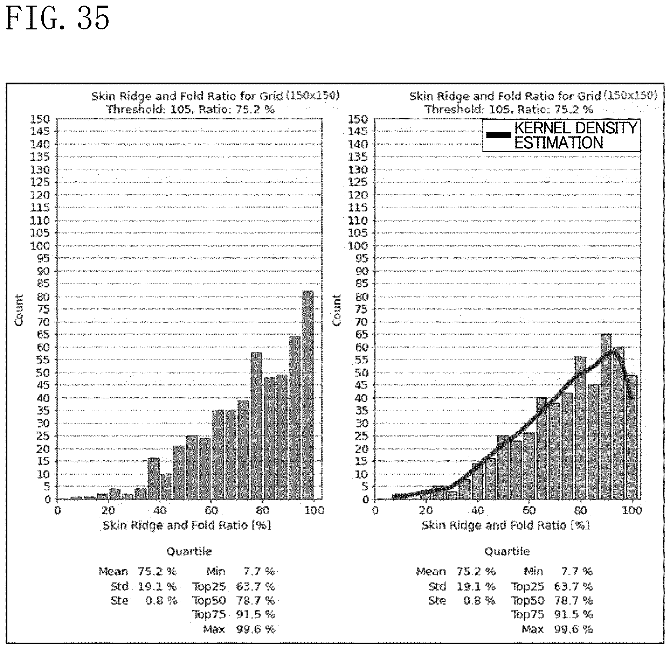

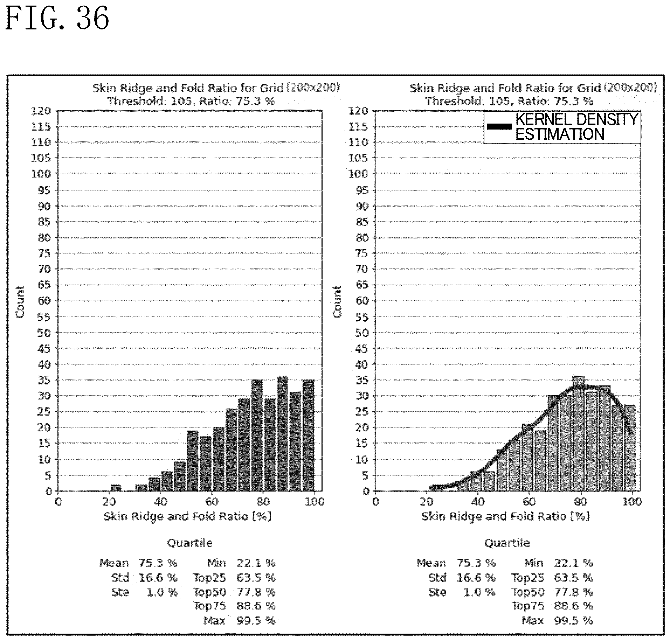

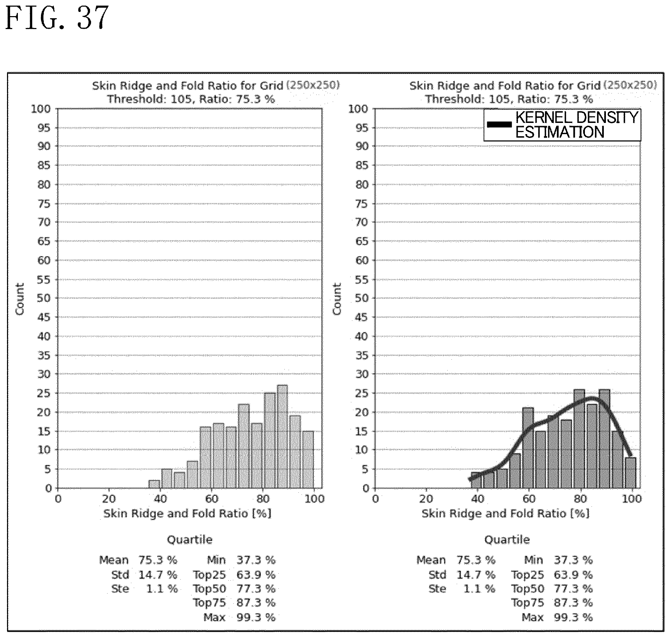

FIGS. 34 to 37 are graphs showing the ratios between skin ridges and skin folds on the forehead of an atopic dermatitis patient, and correspond to FIGS. 26 to 29, respectively. As compared to the graphs of FIGS. 26 to 29 showing the healthy person, a peak is shifted to the right (to a higher ratio between skin ridges and skin folds) as a whole, or a plurality of peaks are found. By viewing these graphs, the difference between the healthy person and the atopic dermatitis patient can be grasped, and the fineness and conditions of the skin of the atopic dermatitis patient can also be grasped. The treatment effects can thus be presented as objective indexes in follow-up observation.

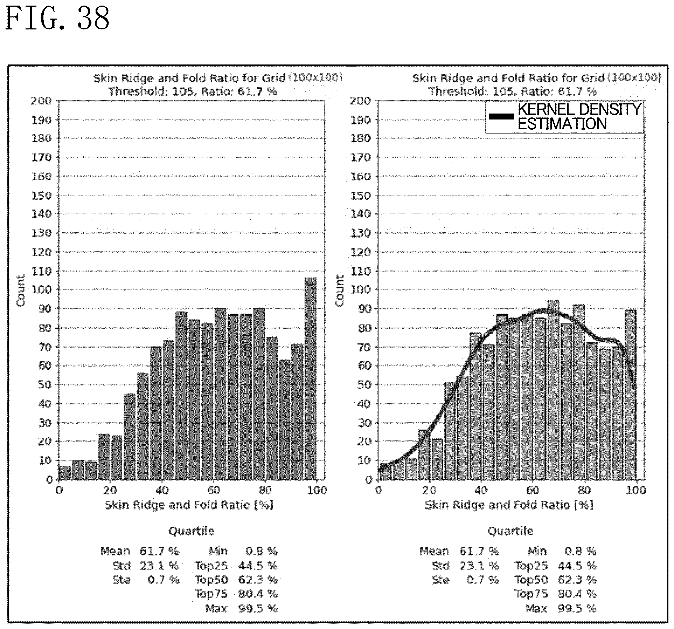

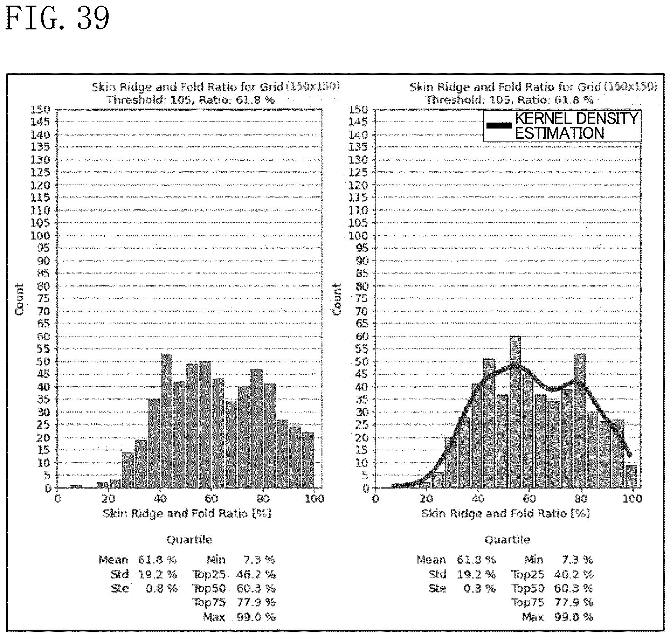

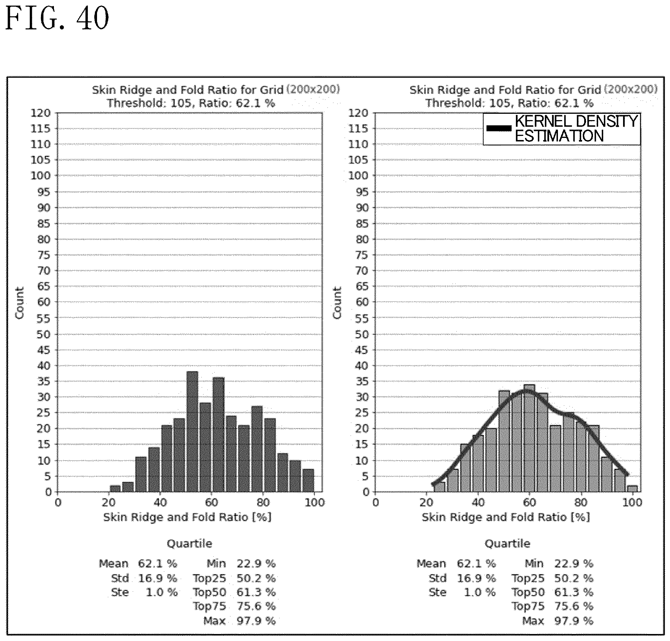

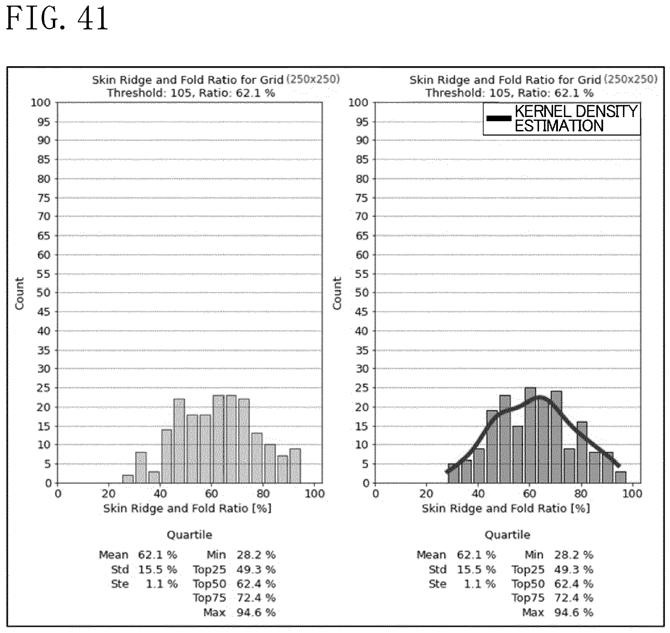

FIGS. 38 to 41 are graphs showing the ratios of skin ridges and skin folds on an elbow of the atopic dermatitis patient, and correspond to FIGS. 26 to 29, respectively. As compared to the graphs of FIGS. 26 to 29 showing the healthy person, a peak is shifted from the center, and a plurality of peaks are found. By viewing these graphs, the difference between the healthy person and the atopic dermatitis patient can be grasped, and the fineness and conditions of the skin of the atopic dermatitis patient can also be grasped. The treatment effects can thus be presented as objective indexes in follow-up observation.

### (Advantageous Effects of Embodiment)

As described above, this embodiment allows generation of a likelihood map image of a skin surface, using the machine learning identifier 24, and allows a skin ridge region and sweat droplets to be distinguished, using the likelihood map image. It is therefore possible to eliminate individual variations in analysis and improve the accuracy in analyzing the conditions of the skin surface, and reduce the time required for the analysis.

The embodiment described above is a mere example in all respects and should not be interpreted as limiting. All modifications and changes belonging to the equivalent scope of the claims fall within the scope of the present invention.

### INDUSTRIAL APPLICABILITY

As described above, the skin surface analysis device and the skin surface analysis method according to the present invention can be used to analyze a human skin surface, for example.

### DESCRIPTION OF REFERENCE CHARACTERS

- 1: Skin Surface Analysis Device
- 20: Image Input Section
- 21: Grayscale Processor
- 22: Image Enhancement Processor
- 23: Patch Image Generator
- 24: Machine Learning Identifier
- 24a: Skin Ridge And Skin Fold Detector
- 24b: Sweat Droplet Detector
- 25: Whole Image Generator
- 26: Likelihood Map Generator
- 27: Binarization Processor
- 28: Region Extractor
- 29: Sweat Droplet Extractor
- 30: Skin Ridge Analyzer
- 31: Sweat Droplet Analyzer
- 100: Transcription Material

## Claims

1. A skin surface analysis device for analyzing a skin surface, using a transcription material to which a human skin surface microstructure is transcribed, the skin surface analysis device comprising:
an image input section to which an image obtained by imaging the transcription material is input;
a local image enhancement processor configured to execute local image enhancement processing of enhancing contrast of a local region of the image input to the image input section to generate an enhanced image;
a patch image generator configured to divide, into a plurality of patch images, the enhanced image generated by the local image enhancement processor;
a machine learning identifier configured to receive the patch images generated by the patch image generator and execute segmentation of each of the patch images received;
a whole image generator configured to generate a whole image by combining the patch images segmented and output from the machine learning identifier;
a likelihood map generator configured to generate a likelihood map image of skin ridges based on a result of the segmentation from the whole image generated by the whole image generator;
a binarization processor configured to execute binarization processing on the likelihood map image generated by the likelihood map generator to generate a binary image;
a region extractor configured to extract a skin ridge region based on the binary image generated by the binarization processor; and
a skin ridge analyzer configured to calculate an area of the skin ridge region extracted by the region extractor.

2. A skin surface analysis device for analyzing a skin surface, using a transcription material to which a human skin surface microstructure is transcribed, the skin surface analysis device comprising:
an image input section to which an image obtained by imaging the transcription material is input;
a local image enhancement processor configured to execute local image enhancement processing of enhancing contrast of a local region of the image input to the image input section to generate an enhanced image;
a patch image generator configured to divide, into a plurality of patch images, the enhanced image generated by the local image enhancement processor;
a machine learning identifier configured to receive the patch images generated by the patch image generator and execute segmentation of each of the patch images received;
a whole image generator configured to generate a whole image by combining the patch images segmented and output from the machine learning identifier;
a likelihood map generator configured to generate a likelihood map image of sweat droplets based on a result of the segmentation from the whole image generated by the whole image generator;
a sweat droplet extractor configured to extract the sweat droplets based on the likelihood map image generated by the likelihood map generator; and
a sweat droplet analyzer configured to calculate a distribution of the sweat droplets extracted by the sweat droplet extractor.

3. The skin surface analysis device of claim 1, further comprising:
a likelihood map generator configured to generate a likelihood map image of sweat droplets based on a result of the segmentation from the whole image generated by the whole image generator;
a sweat droplet extractor configured to extract the sweat droplets based on the likelihood map image generated by the likelihood map generator; and
a sweat droplet analyzer configured to calculate a distribution of the sweat droplets extracted by the sweat droplet extractor.

4. The skin surface analysis device of any one of claims 1 to 3, wherein
the transcription material is obtained by an impression mold technique, and
the skin surface analysis device further comprises a grayscale processor configured to convert an image obtained by imaging the transcription material to grayscale.

5. The skin surface analysis device of any one of claims 1 to 4, wherein
the patch image generator generates the patch images so that adjacent ones of the patch images partially overlap each other.

6. The skin surface analysis device of any one of claims 1 to 5, wherein
an input image and an output image of the machine learning identifier have a same resolution.

7. The skin surface analysis device of claim 1, wherein
the skin ridge analyzer sets, on an image, a plurality of grids in a predetermined size and calculates a ratio between the skin ridge region and a skin fold region in each of the grids.

8. The skin surface analysis device of claim 7, wherein
the skin ridge analyzer converts the ratio between the skin ridge region and the skin fold region in each of the grids into numbers to obtain a frequency distribution.

9. The skin surface analysis device of claim 1, wherein
the region extractor determines, after extracting the skin ridge region, whether each portion of the skin ridge region extracted is raised and divides the skin ridge region by a portion determined to be unraised.

10. The skin surface analysis device of claim 3, further comprising:
an information output section configured to generate and output information on a shape of the skin ridge region extracted by the region extractor.

11. A skin surface analysis method of analyzing a skin surface, using a transcription material to which a human skin surface microstructure is transcribed, the skin surface analysis method comprising:
image input of inputting an image obtained by imaging the transcription material;
local image enhancement processing of executing local image enhancement processing of enhancing contrast of a local region of the image that is input in the image input to generate an enhanced image;
patch image generation of dividing, into a plurality of patch images, the enhanced image generated in the local image enhancement processing;
segmentation of inputting, to a machine learning identifier, the patch images generated in the patch image generation and executing segmentation of each of the patch images input, using the machine learning identifier;
whole image generation of combining the patch images after the segmentation to generate a whole image;
likelihood map generation of generating a likelihood map image of skin ridges based on a result of the segmentation from the whole image generated in the whole image generation;
binarization processing of executing binarization processing on the likelihood map image generated in the likelihood map generation to generate a binary image;
region extraction of extracting a skin ridge region based on the binary image generated in the binarization processing; and
skin ridge analysis of calculating an area of the skin ridge region extracted in the region extraction.

12. A skin surface analysis method of analyzing a skin surface, using a transcription material to which a human skin surface microstructure is transcribed, the skin surface analysis method comprising:
image input of inputting an image obtained by imaging the transcription material;
local image enhancement processing of executing local image enhancement processing of enhancing contrast of a local region of the image that is input in the image input to generate an enhanced image;
patch image generation of dividing, into a plurality of patch images, the enhanced image generated in the local image enhancement processing;
segmentation of inputting, to a machine learning identifier, the patch images generated in the patch image generation and executing segmentation of each of the patch images input, using the machine learning identifier;
whole image generation of combining the patch images after the segmentation to generate a whole image;
likelihood map generation of generating a likelihood map image of sweat droplets based on a result of the segmentation from the whole image generated in the whole image generation;
sweat droplet extraction of extracting the sweat droplets based on the likelihood map image generated in the likelihood map generation; and
sweat droplet analysis of calculating a distribution of the sweat droplets extracted in the sweat droplet extraction.
